# EUROPEAN PATENT SPECIFICATION

(11) **EP 3 821 936 B1**
(45) Date of publication and mention of the grant of the patent: **19.03.2025**
(21) Application number: 20203255.3
(22) Date of filing: 22.10.2020
(51) Int. Cl.: A61M 27/00, A61M 25/04, A61B 17/68, A61B 90/00, A61B 34/20, A61B 5/00, A61B 8/08, A61B 90/10, A61B 8/00, A61B 17/00

(54) **CEREBRAL SPINAL FLUID SHUNT PLUG**
STOPFEN FÜR ZEREBRALSHUNT
BOUCHON DE DÉRIVATION DE FLUIDE SPINAL CÉRÉBRAL

(30) Priority: 24.10.2019 US 201916662624; 16.10.2020 US 202063092606 P
(43) Date of publication of application: 19.05.2021
(73) Proprietor: Longeviti Neuro Solutions LLC, Hunt Valley, MD 21030 (US)
(72) Inventor: CHRISTOPHER, Jesse, Hunt Valley, MD 21030 (US); RABINOVITZ, Bradley, Severna Park, MD 21146 (US); JOHNSON, Todd, Chalfont, PA 18914 (US)
(74) Representative: Glück Kritzenberger Patentanwälte PartGmbB

(56) References cited:
- US-A- 4 281 667
- US-A1- 2016 263 361
- US-A1- 2017 156 596
- US-B1- 7 346 391

## Description

### BACKGROUND OF THE INVENTION

### 1. Field of the Invention

The present invention relates to a cerebral spinal fluid shunt plug.

### 2. Description of the Related Art

Hydrocephalus is a condition in which an excessive accumulation of cerebral spinal fluid is encountered. Cerebral spinal fluid is the clear fluid that surrounds the brain and the spinal cord. The excessive accumulation results in abnormal dilation of the ventricles within the brain. This dilation may cause the accumulation of potentially harmful pressure on the tissues of the brain.

Hydrocephalus is most often treated through the utilization of a shunt system. Cerebral spinal fluid shunt systems divert the flow of cerebral spinal fluid from a site within the ventricles to another area of the body where the cerebral spinal fluid can be absorbed as part of the circulatory system.

Shunt systems are commonly installed by creating a small hole within the skull, commonly referred to as a burr hole. A ventricular catheter is passed through the burr hole and positioned in the ventricular space. A peritoneal catheter is positioned at another location within the body where the cerebral spinal fluid can be diverted and absorbed. For example, it is common to either shunt the cerebral spinal fluid from the cerebral ventricles to the peritoneal cavity for reabsorption into the blood through the peritoneum or the cerebral spinal fluid may be shunted from the cerebral ventricles into the right atrium of the heart where the cerebral spinal fluid is directly shunted into the blood circulation.

In accordance with a typical procedure, incisions are made for the ventricular catheter and the peritoneal catheter. The peritoneal catheter is then positioned, and a burr hole is formed within the skull. Thereafter, the ventricular catheter is positioned. The ventricular catheter and the peritoneal catheter are then connected to a shunt valve which controls the flow of cerebral spinal fluid from the ventricle, through the ventricular catheter, and to the peritoneal catheter. The incisions are then closed.

In addition to common complications, such as shunt malfunction, shunt failure and shunt infection, the utilization of catheters passing through the burr hole with the shunt valve positioned between the skull and the scalp results in other problems. For example, the shunt valve may resorb bone thereby creating a defect in the skull. In addition, the shunt valve and/or ventricular catheter are susceptible to movement. Still further, the ventricular catheter is susceptible to kinks as it passes through and around the burr hole.

US 4 281 667 A discloses a differential pressure sensing device which is fully implanted in the body of a patient to monitor internal pressure such as intracranial pressure. A movable element in the sensor communicates with the internal pressure of the body to be measured on one side and the atmospheric pressure on the other, the latter communicated through the intact skin and a nearly coplanar membrane. Figure 10 of US 4 281 667 shows a shunt plug housing including a shunt valve recess formed therein and a recess with an access hole, a shunt valve shaped and dimensioned for positioning within the shunt valve recess of the shunt plug housing.

US 2016/263361 A1 discloses a ventricular catheter assembly including a proximal catheter and a cooperating cranial cover. The cranial cover includes a base plate having an opening aligned with a burr hole in the skull of a person. A guide extends upwardly from the base plate and receives the proximal catheter.

Finally, US 7 346 391 B1 discloses a cerebral and/or interface system having a housing mechanism configured to be at least partially spaced in a cavity formed in the subject's skull; an attaching mechanism for attaching the housing mechanism to the subject's skull; a sealing mechanism for providing a fluid-tight seal between the housing mechanism and the subject's skull; a control mechanism spaced within the housing mechanism; a communication mechanism with one or more sensors embedded in the subject's brain connecting the control mechanism to the subject's brain; and a power source spaced within the housing mechanism.

With the foregoing in mind, it is desirable to improve upon current techniques for the placement of cerebral spinal fluid shunt systems.

### SUMMARY OF THE INVENTION

The present invention provides a cerebral spinal fluid shunt plug including a shunt plug housing including an upper surface, a lower surface, continuous side walls extending between the upper surface and the lower surface, as well as about the periphery of the shunt plug housing, a shunt valve recess being formed in the upper surface of the shunt plug housing and either an intracranial monitoring recess with an access hole or a window recess with a central access hole formed in the upper surface of the shunt plug housing, the shunt plug housing further including passageways allowing the shunt valve recess to communicate with an exterior of the shunt plug housing, the access holes or passageways being shaped and dimensioned to allow for connection of a ventricular catheter and a peritoneal catheter with the shunt valve housed within the recess of the shunt plug housing, the housing being shaped and dimensioned for positioning within a physician formed cranial hole in a manner allowing the upper surface of the housing to be substantially flush with an outer surface of a skull and a projection along the lower surface being positioned within the cranial hole; and a window or an intracranial monitoring device shaped and dimensioned for respectively positioning within either the window recess or the intracranial monitoring recess of the shunt plug housing.

In some embodiments, the recess with an access hole is a window recess with an access hole and the cerebral spinal fluid shunt plug includes the window shaped and dimensioned for positioning within the window recess.

**In** some embodiments, the cerebral spinal fluid shunt plug further includes an intracranial monitoring device recess with an access hole and an intracranial monitoring device shaped and dimensioned for the passage through the access hole of the intracranial monitoring device recess.

**In** some embodiments, the central access hole extending from the window recess to a lower surface of the shunt plug housing is shaped and dimensioned for the passage of light, sound, and/or radio waves therethrough so as to access the brain for imaging and treatment.

**In** some embodiments, the window is optically transparent.

**In** some embodiments, the window is optically translucent to all light waves.

In some embodiments, the window is sonolucent.

In some embodiments, the window is radiolucent.

In some embodiments, the window is optically transparent, optically translucent to all light waves, is sonolucent, and is radiolucent.

In some embodiments, the window comprises polymethyl methacrylate (PMMA).

In some embodiments, the window is a lucent disk.

In some embodiments, the lucent disk includes an upper surface and a lower surface and the curvature of the upper surface differs from the curvature of the lower surface.

In some embodiments, the lucent disk includes an alignment feature.

In some embodiments, the alignment feature includes a series of markings at different depths within the lucent disk.

In some embodiments, the series of markings includes an outer first lucent disk marking and an inner second lucent disk marking formed along the upper and lower surfaces, respectively, of the lucent disk.

In some embodiments, the series of markings further includes an interior lucent disk marking formed within a body of the lucent disk and in alignment with the outer first lucent disk marking and an inner second lucent disk marking.

In some embodiments, the lucent disk includes channels.

In some embodiments, the intracranial monitoring device is a wireless intracranial monitoring device.

In some embodiments, the intracranial monitoring device includes a probe that passes through the access hole.
Other advantages of the present invention will become apparent from the following detailed description when viewed in conjunction with the accompanying drawings, which set forth certain embodiments of the invention.

### BRIEF DESCRIPTION OF THE DRAWINGS

Figure 1 is a perspective view of a shunt plug housing for a shunt plug (not part of the present invention).
Figure 2 is an exploded view of the shunt plug housing shown in Figure 1.
Figure 3 is a perspective view of the shunt plug housing (not part of the present invention) with alternate positions for the access apertures.
Figures 4, 5, and 6 are sectional views of the shunt plug, including the shunt plug housing, the shunt valve, and the catheters, wherein the top portion of the shunt plug housing has been cut away to more clearly show the shunt valve (not part of the present invention).
Figure 7 is an exploded representative view of the installation process.
Figure 8 is an exploded representative view of the installation process with a shunt plug of an alternate shape.
Figures 9, 10, and 11 are perspective views of a shunt plug housing in accordance with an alternate embodiment (not part of the present invention) and showing three different cavity shapes for accommodating different shunt valves.
Figure 12 is a perspective view of a cerebral spinal fluid shunt plug in accordance with the embodiment shown with reference to Figures 9, 10, and 11.
Figures 13 to 18 show the steps associated with the implantation of a cerebral spinal fluid shunt plug in accordance with the embodiment disclosed with reference to Figures 9, 10, and 11.
Figures 19, 20, 21, and 22 are perspective views of alternate embodiments of a cerebral spinal fluid shunt plug (not part of the present invention).
Figures 23 and 24 are top and bottom perspective views showing a cerebral spinal fluid shunt plug in accordance with the present invention.
Figure 25 is a schematic showing the process of implanting the cerebral spinal fluid shunt plug shown in Figures 23 and 24.
Figures 26 and 27 are perspective views of a cerebral spinal fluid shunt plug in accordance with an alternate embodiment of the present invention with the lucent disk shown and not shown, respectively, and also showing that the lucent disk and window recess may be formed in various shapes.
Figure 28 is a perspective view of a cerebral spinal fluid shunt plug in accordance with an alternate embodiment of the present invention.
Figure 29 is a perspective views of a cerebral spinal fluid shunt plug in accordance with an alternate embodiment of the present invention.
Figures 30, 31, and 32 are respectively a cross sectional view, a perspective view, and a cross sectional view showing various embodiment of a lucent disk.

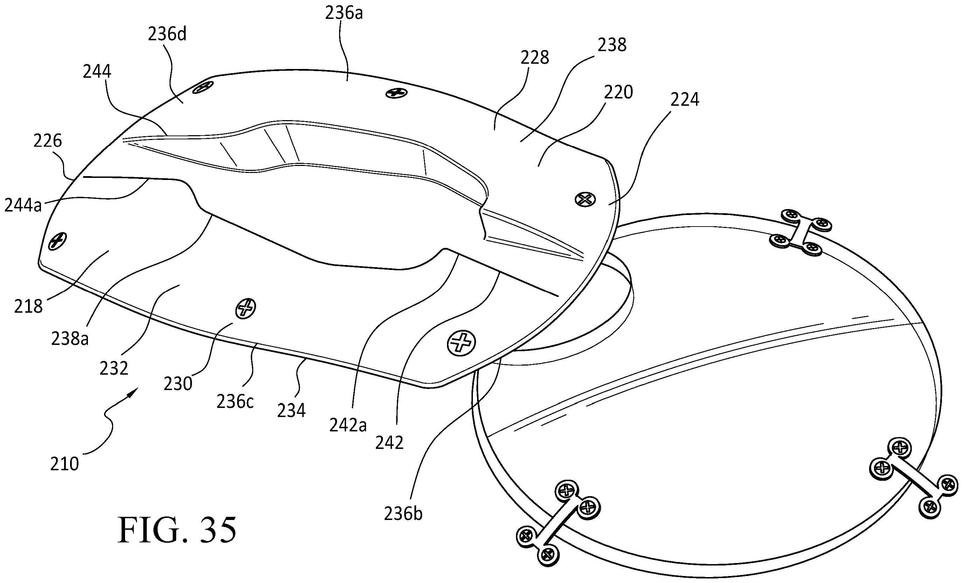

Figures 33, 34, and 35 are perspective views of a cerebral spinal fluid shunt plug assembly in accordance with alternate embodiments of the present invention.
Figures 36, 37, and 38 are perspective views of a cerebral spinal fluid shunt plug assembly in accordance with further embodiments (not part of the present invention).

### DESCRIPTION OF THE PREFERRED EMBODIMENTS

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as a basis for teaching one skilled in the art how to make and/or use the invention.

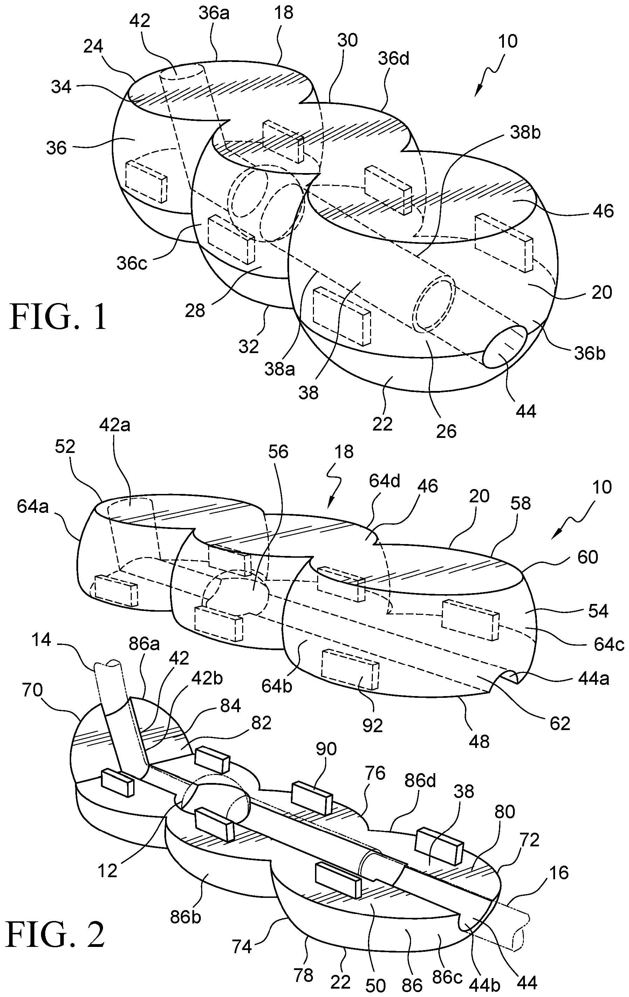

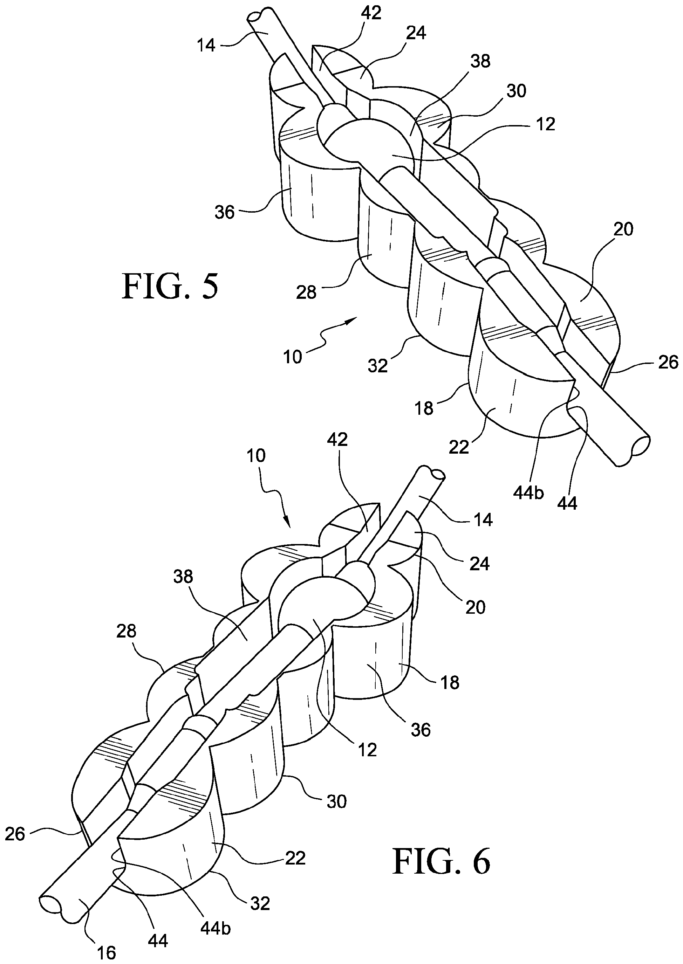

Referring to Figures 1 to 7 (not part of the present invention), various embodiments of a cerebral spinal fluid shunt plug 10 are disclosed in accordance with the present invention. It should be appreciated similar reference numerals are used for the various different embodiments. The shunt plug 10 is shaped and dimensioned for positioning within a physician formed cranial hole 100. The shunt plug 10 is further shaped and dimensioned for housing a shunt valve 12 in a reliable and secure manner so that a ventricular catheter 14 and peritoneal catheter 16 may be positioned without fear that the shunt valve 12 might move and/or the catheters 14, 16 might become disengaged from their desired locations.

The shunt plug 10 includes a shunt plug housing 18 composed of a bottom first housing member 20 and a top second housing member 22. The bottom first housing member 20 and top second housing member 22 are shaped and dimensioned for mating so as to define the shunt plug housing 18 in which the shunt valve 12 is positioned. In accordance with the disclosed embodiments, the shunt valve 12 will be placed within the shunt plug housing 18, so as to create the shunt plug 10 of the present invention, at the time of surgery. Further, in accordance with a disclosed embodiment the shunt plug housing is made of HDPE, although it is appreciated other materials may be used without departing from the spirit of the present invention.

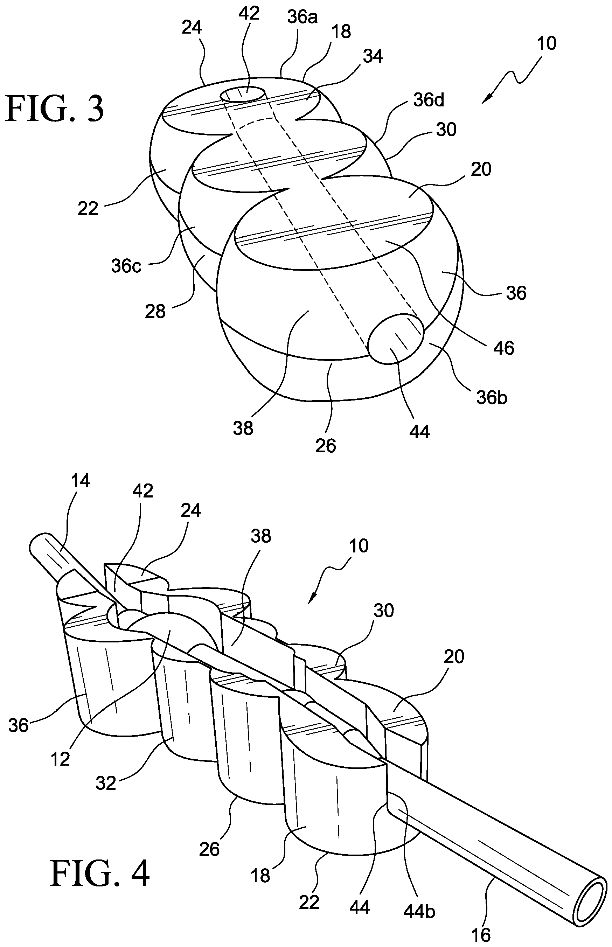

The shunt plug housing 18, when the first housing member 20 and the second housing member 22 are connected together as shown with reference to Figures 1 and 3, includes a first end 24, a second end 26, a first lateral side 28, and a second lateral side 30. The shunt plug housing 18 also includes an upper surface 32, a lower surface 34, and a continuous side wall 36 extending between the upper surface 32 and the lower surface 34, as well as about the periphery of the shunt plug housing 18.

In accordance with the disclosed embodiments, and considering the procedure discussed below in greater detail, the shunt plug housing 18 is structured with consecutive and overlapping cylinders (for example, three cylinders as shown with reference to Figures 1, 2, and 3 and six cylinders as disclosed with reference to Figures 4, 5, and 6). While a shape in accordance with the disclosed embodiment is disclosed herein for the purpose of explaining the present invention, it is appreciated various shapes may be employed within the spirit of the present invention. As will be appreciated after reading the installation procedure presented below, the consecutive and overlapping cylinder structure was selected as a means of optimizing the installation procedure based upon the utilization of a single trephine to create consecutive and overlapping burr holes that ultimately define the cranial hole 100 in which the shunt plug 10 is positioned. As such, the shape of the shunt plug and the mechanism for the creation of the cranial hole are intimately related and may be varied based upon various needs and requirements.

Considering the embodiment disclosed with reference to Figures 1, 2, and 3 (not part of the present invention), the side wall 36 of the disclosed embodiment is formed with a scalloped shape wherein a plurality of arcuate segments extends about the periphery of the shunt plug housing 18. The arcuate segments 36a, 36b at the first end 24 and the second end 26 of the shunt plug housing 18 define an arcuate surface of approximately 220 degrees to 320 degrees, and the arcuate segments 36c, 36d located at the center of the side wall 36 along the first and second lateral sides 28, 30 define an arc of approximately 40 to 140 degrees. In accordance with a preferred embodiment, the outer surface of the side wall 36 may be bowed outwardly so as to define a convex outer surface.

The embodiment disclosed with reference to Figures 4, 5, and 6 (not part of the present invention) is similarly shaped, but includes additional arcuate surfaces requiring the formation of additional burr holes during the installation process. In particular, the shunt plug 10 disclosed in Figures 4, 5, and 6 would require the formation of six burr holes in a highly specific pattern. The pattern employed creates a larger area for accommodating the needs of shunt valves having larger dimensions.

A recess 38 is formed within the shunt plug housing 18. The recess 38 is defined by recessed surfaces 38a, 38b formed along the surfaces of the first housing member 20 and the second housing member 22. The recess 38 is in communication with the exterior of the shunt plug housing 18 via access holes 42, 44 extending from the exterior surface of the shunt plug housing 18 to the recess 38. As will be explained below in greater detail, these access holes 42, 44 allow for connection of the ventricular catheter 14 and the peritoneal catheter 16 with the shunt valve 12 housed within the recess 38 of the shunt plug housing 18. As with the recess 38, the access holes 42, 44 are defined by recessed surfaces 42a, 42b, 44a, 44b formed along the surfaces of the first housing member 20 and the second housing member 22. Depending upon the shape of the shunt plug housing 18 and the shunt valve 12 to be positioned therein, the position of the access holes 42, 44 may be varied to optimize the ultimate positioning of the peritoneal catheter 16 and the ventricular catheter 14 (see, for example, Figures 1 and 3).

In particular, and with reference to Figure 2, the first housing member 20 includes an exterior surface 46 that defines the exterior surface of the shunt plug housing 18 when the first and second housing members 20, 22 are connected together (as shown in Figure 1) to form the complete shunt plug housing 18. The first housing member 20 also includes a mating surface 48 that engages the mating surface 50 of the second housing member 22 when the first and second housing members 20, 22 are connected together to form the complete shunt plug housing 18.

With this in mind, the first housing member 20 includes a first end 52, a second end 54, and first and second lateral sides 56, 58. The first housing member 20 also includes a top surface 60 defining the lower surface 34 of the shunt plug housing 18, a lower surface 62 that forms part of the mating surface 48 of the first housing member 20 that mates with the mating surface 50 of the second housing member 22 so as to define the junction of the first housing member 20 and the second housing member 22, as well as the recess 38 in which the shunt valve 12 is positioned. The first housing member 20 also includes side walls 64a-d extending between the top surface 60 and the lower surface 62. The side walls 64b, 64c, 64d at the first and second lateral sides 56, 58, as well as the second end 54, of the first housing member 20 define a portion of the exterior surface of the shunt plug housing 18. The side wall 64a at the first end 52 of the first housing member 20 forms part of the mating surface 48 of the first housing member 20 that mates with the mating surface 50 of the second housing member 22 so as to define the junction of the first housing member 20 and the second housing member 22.

The second housing member 22 includes a first end 70, a second end 72, and first and second lateral sides 74, 76. The second housing member 22 includes a top surface 78 defining the upper surface 32 of the shunt plug housing 18, a lower surface 80 that forms part of the mating surface 50 of the second housing member 22 that mates with the mating surface 48 of the first housing member 20 so as to define the junction of the first housing member 20 and the second housing member 22, as well as the recess 38 in which the shunt valve 12 is positioned. The second housing member 22 also includes an upwardly directed wall portion 82 extending upwardly from the lower surface 80 at the first end 70 of the second housing member 22. The wall portion 82 includes an interior surface 84 forming part of the mating surface 50 of the second housing member 22 that mates with the mating surface 48 of the first housing member 22 so as to define the junction of the first housing member 20 and the second housing member 22. In particular, the interior surface 84 of the wall portion 82 is shaped and dimensioned to mate with the side wall 64a at the first end 52 of the first housing member 20. The surface of the wall portion 82 opposite the interior surface 84 forms part of the side wall of the shunt plug housing 18 at the first end 24 of the shunt plug housing 18.

The second housing member 22 also includes side walls 86a-d extending between the lower surface 80 and the top surface 78. The side walls 86a-d at the first and second lateral sides 74, 76, as well as the first and second ends 70, 72, of the second housing member 22 define a portion of the exterior surface of the shunt plug housing 18.

Mating of the first housing member 20 with the second housing member 22 is further facilitated by the provision of protrusions 90 along the lower surface 80 of the second housing member 22 and matingly shaped indentations 92 along the lower surface 62 of the first housing member 20.

As discussed above, the recess 38 in which the shunt valve 12 is positioned, as well as the access holes 42, 44 for the passage of the ventricular and peritoneal catheters 14, 16, is formed within the shunt plug housing 18. The recess 38 and access holes 42, 44 are defined by recessed surfaces 38a, 38b, 42a, 42b, 44a, 44b formed along the surfaces of the first housing member 20 and the second housing member 22. In particular, the recessed surfaces 38a, 38b, 42a, 42b defining the recess 38 and the first access hole 42 are formed along the lower surface 80 of the second housing member 22 and the lower surface 62 of the first housing member 20. The recessed surfaces 44a, 44b defining the second access hole 44 are formed along the side wall 64a of the first housing member 20 at the first end 52 thereof and along the interior surface 84 of the wall portion 82 at the first end 70 of the second housing member 22.

With the foregoing description of the first housing member and the second housing member in mind, it is appreciated that the first and second housing members may take various shapes depending upon the desired inter-engagement of these two members when the shunt plug housing is fully formed and ready for use.

As briefly discussed above, the recess 38 defined within the shunt plug housing 18 is shaped and dimensioned for placement of the shunt valve 12 therein. As those skilled in the art will appreciate, a variety of shunt valves are known in the art and the present shunt plug housing 18 may be adapted to accommodate a variety of these shunt valves. For example, the following shunt valves may be used in conjunction with the present invention: CODMAN^{®}/Integra HAKIM^{®} and Certas Programmable Shunt Valve, MEDTRONIC^{®} STRATA^{®}, SOPHYSA^{®} POLARIS^{®}, Ascuelap proGAV^{®}, and INTEGRA^{®} OSV II^{®}. The present invention may also be used in conjunction with the Rickam reservoir and other similar reservoirs used in cerebral spinal fluid management. In accordance with a preferred embodiment, the shunt plug housing 18 should have a surface area along its upper surface 32 of at least five cm² so as to accommodate various shunt valves and to provide the necessary space for placement of the shunt valve 12 within the recess 38 defined within the shunt plug housing 18.

Once the shunt valve 12 is positioned between the first housing member 20 and the second housing member 22 within the recess 38 defined thereby, the first housing member 20 may be connected to the second housing member 22 so as to fully enclose the shunt valve 12 therein. Thereafter, the shunt plug 10 of the present invention may be utilized for the purpose of performing a cerebral spinal fluid shunt procedure.

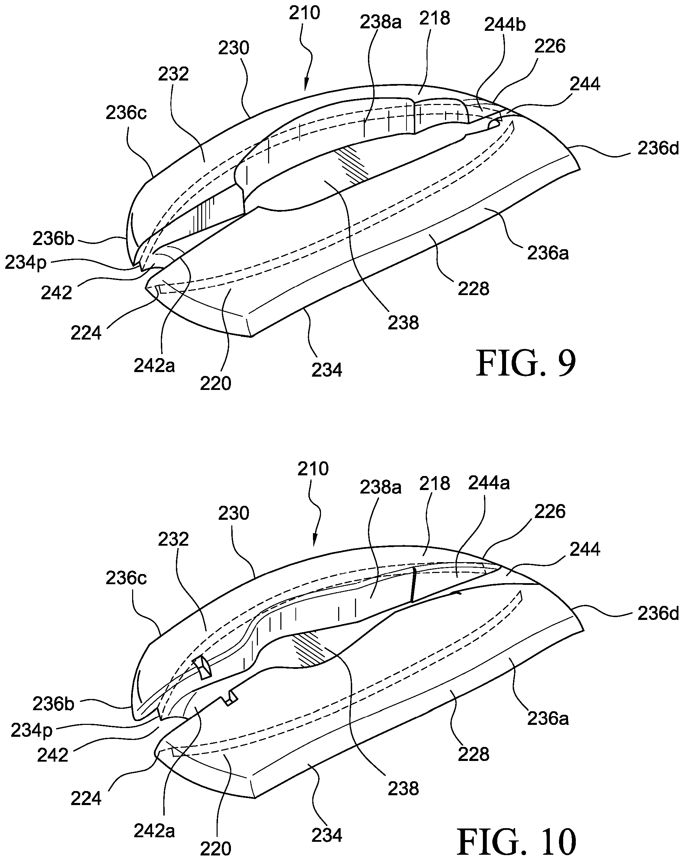

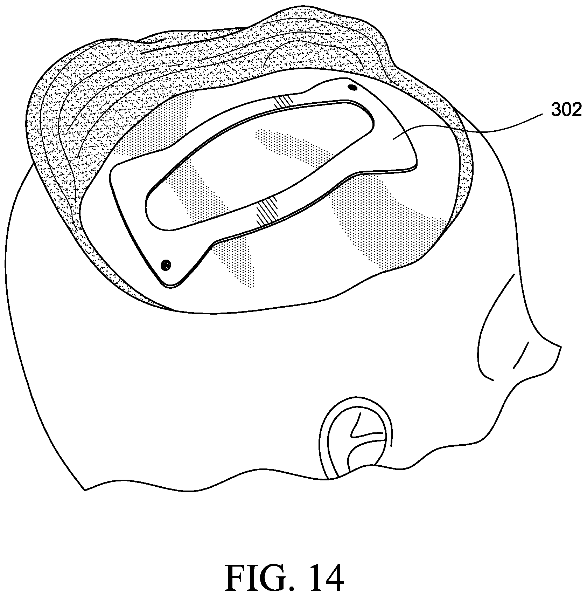

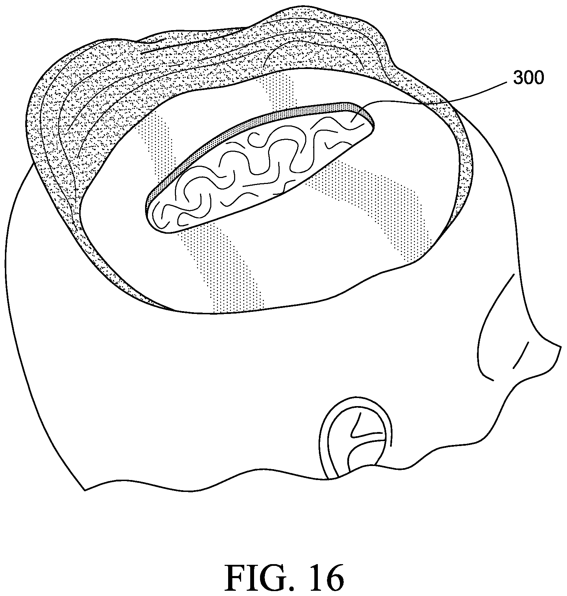

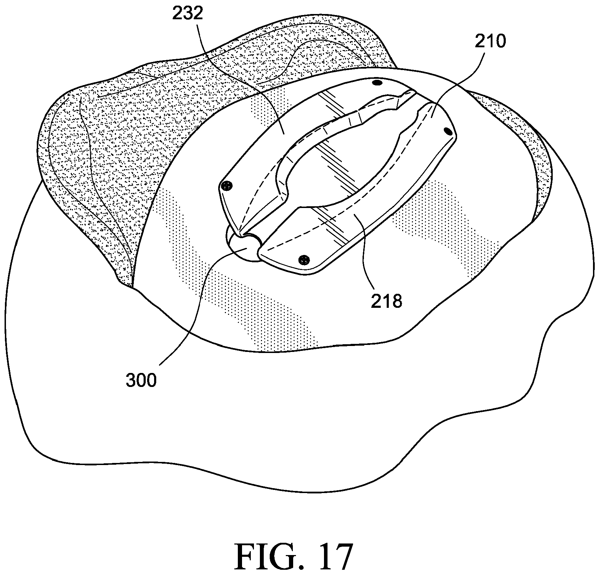

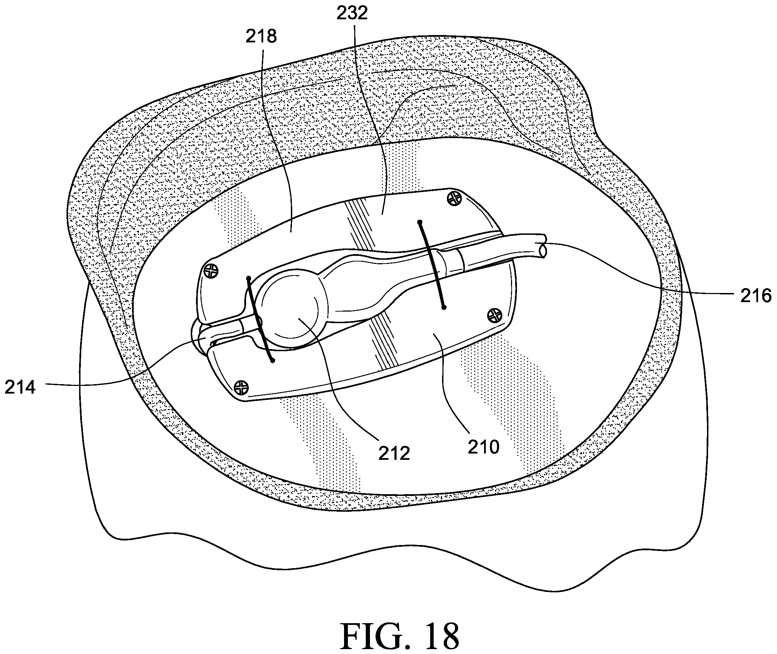

In accordance with yet another embodiment (not part of the present invention) as shown with reference to Figures 9 to 18 (which shows this embodiment in various shapes to accommodate shunt valves from various manufacturers), the shunt plug is structured such that the shunt valve is uncovered. In particular, and as with the previous embodiment, the shunt plug 210 is shaped and dimensioned for housing a shunt valve 212 in a reliable and secure manner so that a ventricular catheter 214 and peritoneal catheter 216 may be positioned without fear that the shunt valve 212 might move and/or the catheters 214, 216 might become disengaged from their desired locations.

The shunt plug 210 includes a shunt plug housing 218 composed of a bottom first housing member 220. In accordance with the disclosed embodiments, the shunt valve 212 will be placed within the shunt plug housing 218, so as to create the shunt plug 210 of the present invention, at the time of surgery.

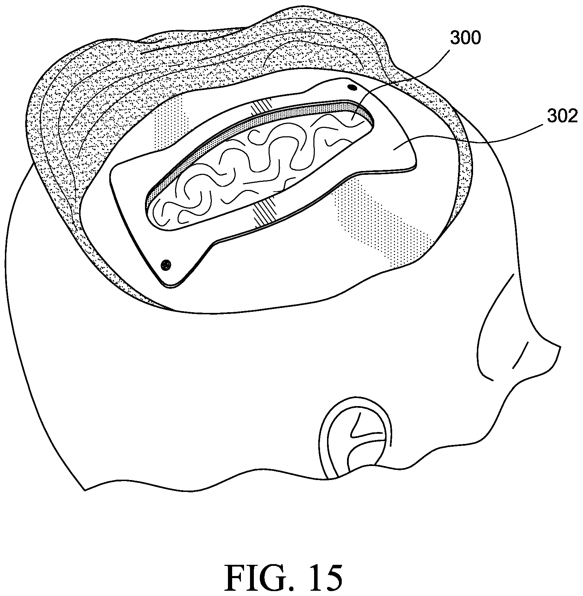

The shunt plug housing 218 includes a first end 224, a second end 226, a first lateral side 228, and a second lateral side 230. The shunt plug housing 218 also includes an upper surface 232, a lower surface 234, and continuous side walls 236a-d extending between the upper surface 232 and the lower surface 234, as well as about the periphery of the shunt plug housing 218. As will be appreciated based upon the following disclosure, the lower surface 234 is provided with a projection 234p that ultimately fits within the cranial hole 300 to assist in holding the shunt plug 210 in position after installation. With this in mind, the projection 234p is elliptically shaped to fit within the cranial hole 300 as shown in Figure 15.

While particular shapes of the shunt plug housing 218 in accordance with the disclosed embodiment are disclosed herein for the purpose of explaining the present invention, it is appreciated various shapes may be employed within the spirit of the present invention. As such, the shape of the shunt plug and the mechanism for the creation of the cranial hole are intimately related and may be varied based upon various needs and requirements. For example, and in contrast with the embodiments described above with reference to Figures 1 to 6, the shunt plug housing includes a substantially elliptical shape.

A recess 238 is formed within the upper surface 232 of the shunt plug housing 218. The recess 238 is in communication with the exterior of the shunt plug housing 218 via access passageways 242, 244 extending from the exterior surface of the shunt plug housing 218 to the recess 238. As will be explained below in greater detail, these access holes (or passageways) 242, 244 allow for connection of the ventricular catheter 214 and the peritoneal catheter 216 with the shunt valve 212 housed within the recess 238 of the shunt plug housing 218. The access passageways 242, 244 are defined by recessed surfaces formed along the upper surface 232 of the shunt plug housing 218. Depending up the shape of the shunt plug housing 218 and the shunt valve 212 to be positioned therein, the position of the access holes (or passageways) 242, 244 may be varied to optimize the ultimate positioning of the peritoneal catheter 216 and the ventricular catheter 214.

As discussed above, the recess 238 in which the shunt valve 212 is positioned, as well as the access holes 242, 244 for the passage of the ventricular and peritoneal catheters 214, 216, is formed within the shunt plug housing 218. The recess 238 and access holes 242, 244 are defined by recessed surfaces 238a, 242a, 244a formed along the upper surface 232 of the shunt plug housing 218. In particular, the recessed surface 238a defining the recess 238 is formed along the upper surface 232 of the shunt plug housing 218; the recessed surface 242a defining the first access hole (or passageway) 242 is formed along the upper surface 232 adjacent the first end 224; and the recessed surfaces 244a defining the second access hole (or passageway) 244 are formed along the side wall 264a of the shunt plug housing 218 at the second end 226 thereof.

As briefly discussed above, the recess 238 defined within the shunt plug housing 218 is shaped and dimensioned for placement of the shunt valve 212 therein. As those skilled in the art will appreciate, and as explained above in conjunction with the prior embodiment, a variety of shunt valves are known in the art and the present shunt plug housing 218 may be adapted to accommodate a variety of these shunt valves. The present invention may also be used in conjunction with the Rickam reservoir and other similar reservoirs used in cerebral spinal fluid management. In accordance with a preferred embodiment, the shunt plug housing 218 should have a surface area along its upper surface 232 of at least five cm² so as to accommodate various shunt valves and to provide the necessary space for placement of the shunt valve 212 within the recess 238 defined within the shunt plug housing 218.

Once the shunt valve 212 is positioned within the recess 238 of the shunt plug housing 218 the shunt plug 210 of the present invention may be utilized for the purpose of performing a cerebral spinal fluid shunt procedure.

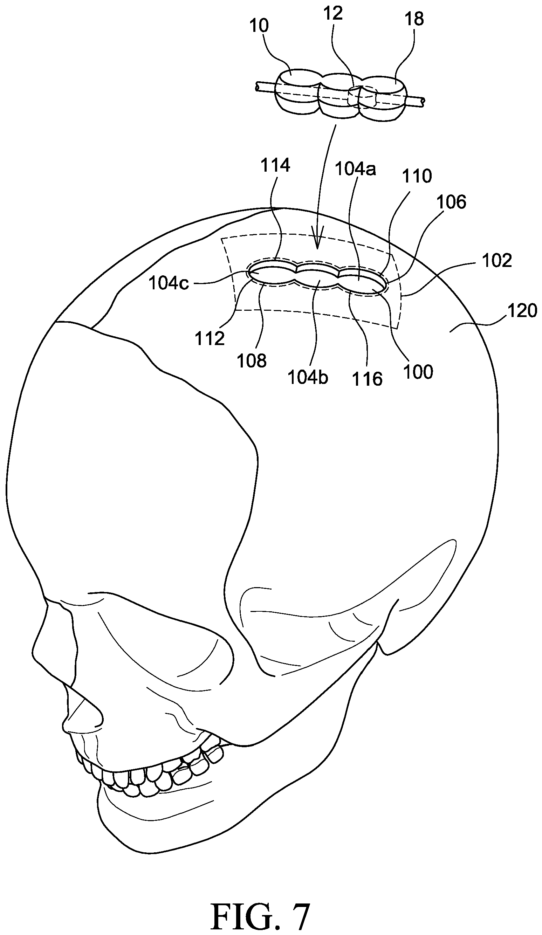

Referring to Figure 7 (not part of the present invention), and with particular reference to the embodiment disclosed in Figures 1, 2, and 3, the procedure is first initiated by making the required incision for passage of the peritoneal catheter 16. Thereafter, a cranial incision is made and the cranial hole 100 is created utilizing a template 102 (shown in broken lines) and predefined trephine (not shown). In contrast to prior art procedures, a singular burr hole is not formed. Rather, adjacent circular holes 104a-c (for example, three as shown in the disclosed embodiment) are formed creating the cranial hole 100 that is shaped for snuggly fitting the shunt plug 10 therein. As with the shape of the shunt plug 10, the cranial hole 100 created in accordance with the present invention includes scalloped edges. In particular, the cranial hole 100 includes first and second ends 106, 108 with an arcuate surface 110, 112 of approximately 220 to 320 degrees, as well as first and second lateral arcuate surfaces 114, 116 of approximately 40 to 140 degrees. Given the matching shape of the cranial hole 100 and the shunt plug 10, the shunt plug 10 will fit snugly within the cranial hole 100 thereby minimizing potential movement after completion of the procedure.

With the cranial hole 100 completed, the ventricular catheter 14 is positioned within the ventricle and the peritoneal catheter 16 is positioned with the body as using well know medical procedures. Thereafter, the ends of the peritoneal catheter 16 and the ventricular catheter 14 adjacent the shunt plug 10 may be secured to the shunt valve 12 housed within the shunt plug 10 by passing the ends of the respective catheters into the first and second access holes 42, 44 formed at locations along the exterior of the shunt plug housing 18. Thereafter, the shunt plug 10 is positioned within the cranial hole 100. The shunt plug 10 is mounted within the cranial hole 100 such that the upper surface 32 is substantially flush with the outer surface of the skull 120. It is, however, appreciated the exact positioning of the shunt plug will vary based upon specific anatomical characteristics of the patient. Once the shunt plug 10 is properly positioned, the shunt valve 12 is actuated utilizing well known procedures, and the procedure is completed in accordance with known medical procedures.

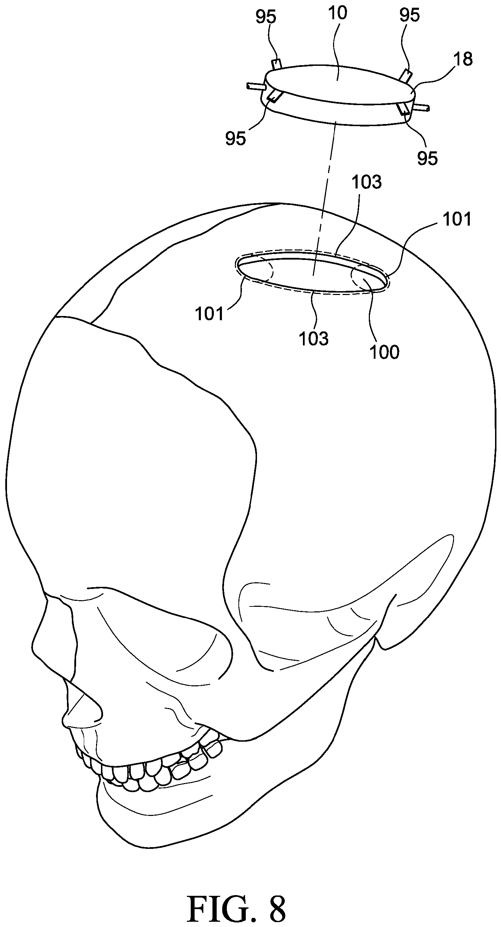

As discussed above, the shunt plug of the present invention may take various shapes. One possible shape where simplicity is considered to be important might involve an elliptically shaped shunt plug housing 18 as shown with reference to Figure 8. Such an embodiment would require a cranial hole 100 formed by the creation of two burr holes 101 with two connecting cuts 103 made in the shape of the shunt plug 10 so as to allow for placement of the shunt plug 10 within the cranial hole 100. It is, however, appreciated the cranial hole may be made using any method of creating an elliptical craniectomy acceptable by those skilled in the art. When using such an embodiment, it is appreciated variations in the cuts 103 between the two burr holes 101 are likely and the shunt plug 10 is therefore provided with a plurality of attachment tabs 95 that may be used to secure the shunt plug 10 to the area of the skull 120 immediately adjacent to the cranial hole 100.

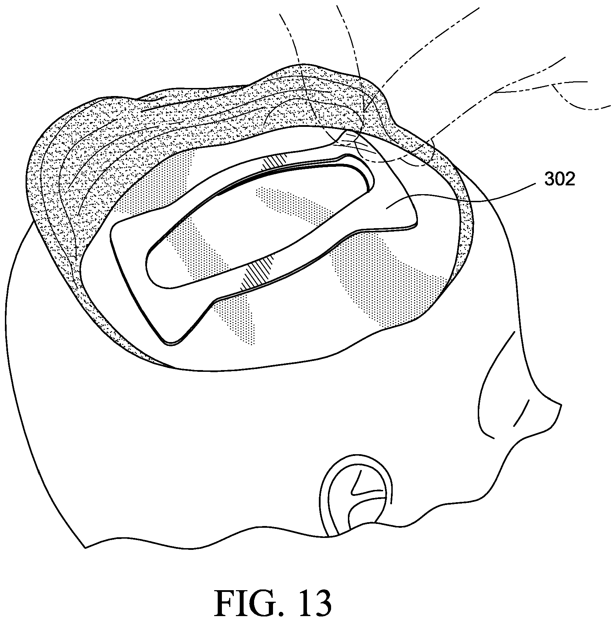

Referring to Figures 13 to 18 (not part of the present invention), and with particular reference to the embodiment disclosed in Figures 9 to 18 (not part of the present invention), the procedure is first initiated by making the required incision for passage of the peritoneal catheter 216. Thereafter, a cranial incision is made and the cranial hole 300 in the skull 320 is created utilizing a template 302 (shown in broken lines). In accordance with a preferred embodiment, and considering the elliptical shape of the cranial hole 300, burr holes are formed at the respective ends of the template 302, and the remainder of the skull 320 is cut away along the lines as defined by the template 302. As with the prior embodiment, it is appreciated the cranial hole may be made using any method of creating an elliptical craniectomy acceptable by those skilled in the art. Given the matching shape of the cranial hole 300 and the shunt plug 210, the shunt plug 210 will fit snugly within the cranial hole 300 thereby minimizing potential movement after completion of the procedure.

With the cranial hole 300 completed, the ventricular catheter 214 is positioned within the ventricle and the peritoneal catheter 216 is positioned with the body as using well know medical procedures. Thereafter, the shunt plug housing 218 is positioned within the cranial hole 300 with the upper surface 232 facing upwardly, and the ventricular catheter 214 is cut to an appropriate length. The ends of the peritoneal catheter 216 and the ventricular catheter 214 adjacent the shunt plug 210 are then secured to the shunt valve 212 and the shunt valve 212 is positioned within the shunt plug housing 218. In particular, the shunt plug 210 is mounted within the cranial hole 300 such that the upper surface 232 is substantially flush with the outer surface of the skull 320 and the projection 234p along the lower surface 234 is positioned within the cranial hole 300. As such, portions along the periphery of the shunt plug housing 218 overlie the skull 320, and screws may be passed therethrough to facilitate secure attachment of the shunt plug 210 to the skull. It is, however, appreciated the exact positioning of the shunt plug will vary based upon specific anatomical characteristics of the patient. Once the shunt plug 210 is properly positioned and secured in place using known techniques, the shunt valve 212 is actuated utilizing well known procedures, and the procedure is completed in accordance with known medical procedures.

With the foregoing in mind, the present shunt plug offers multiple advantages. It eliminates mobility of the shunt valve and/or reservoir. As a result, the shunt valve location is known and will not migrate caudal, cephalad, anterior, or posterior which can cause challenges during revision surgery. The ventricular catheter is a precise distance from the shunt valve to the ventricle, therefore mobility of the shunt valve can displace the location of the ventricular catheter. The present shunt plug eliminates cranial deformity as it avoids the need to implant the shunt valve on top of the cranium and underneath the scalp. As a result, pressure on the scalp is minimized along with any accompanying complications such as pain or implant extrusion. In addition, the present shunt plug minimizes micro-motion; that is, the well documented fact that implant micro-motion can lead to bone resorption (causing further deformity) and can lead to infection. Finally, the present shunt plug minimizes catheter kinking as the sharpest angle in the catheters pathway is the top of the perforator made burr hole and by controlling the angle of entry of the catheter, the risk of occlusion is minimized.

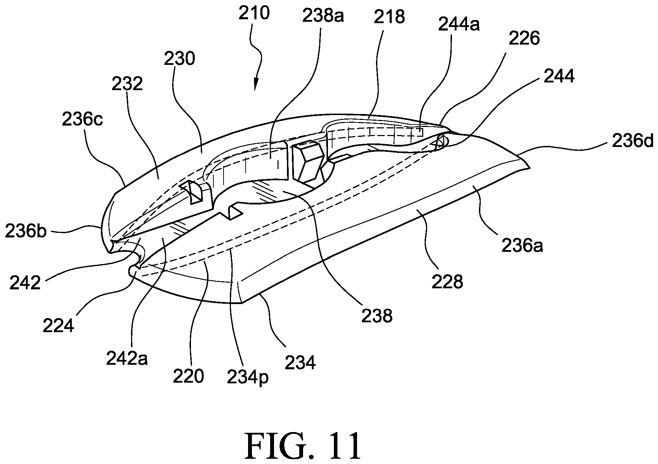

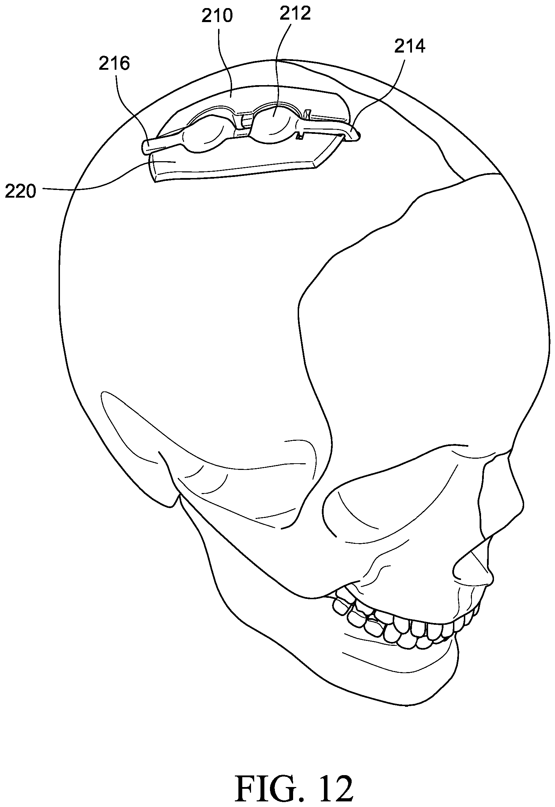

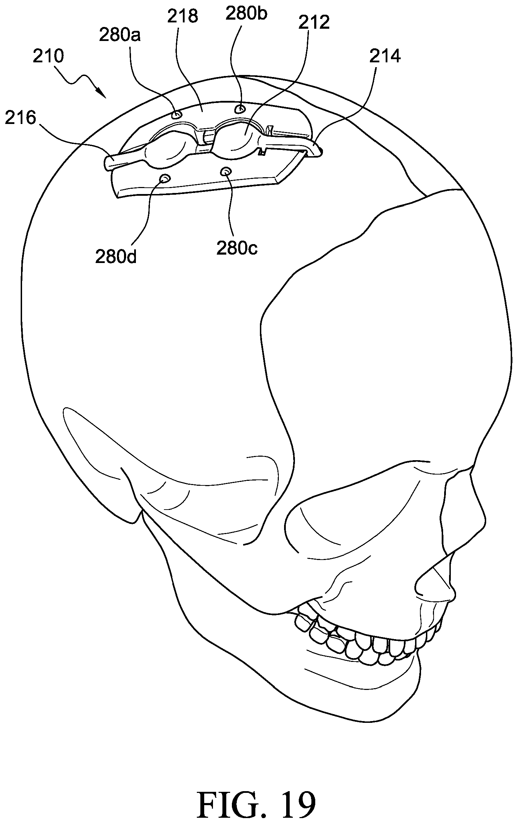

It is appreciated that once the cerebral spinal fluid shunt plug of the present invention is implanted within the cranium and covered by the scalp, it may be desirable to identify, for example, via triangulation, a specific point or points on the cerebral spinal fluid shunt plug, in particular, the shunt valve itself; for example, to identify and/or locate the center of the programmable portion of the programmable shunt valve or reservoir. To achieve this, and with reference to Figures 19 to 22 (not part of the present invention), various embodiments are disclosed. It is appreciated these variations are disclosed in accordance with the embodiment described with reference to Figures 11 and 12 (not part of the present invention), and the variations described herein may be applied to any of the embodiments disclosed herein.

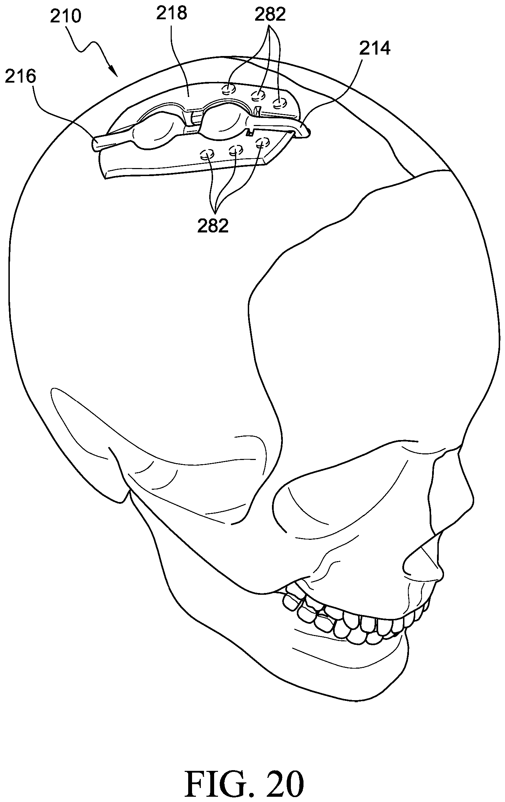

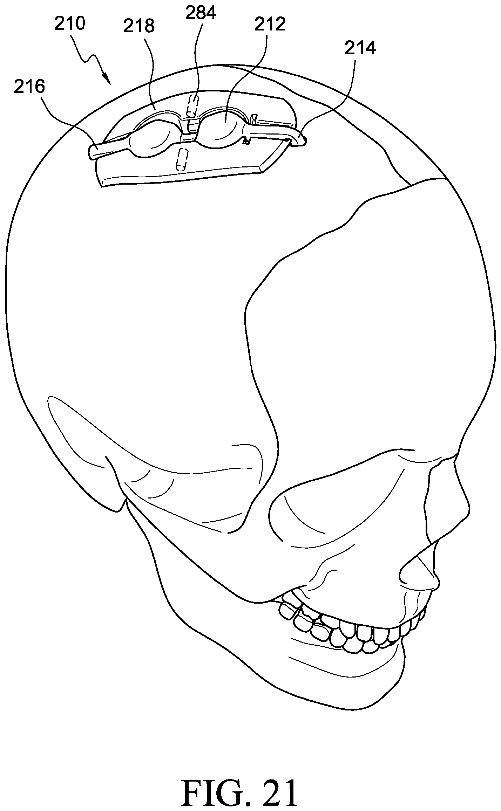

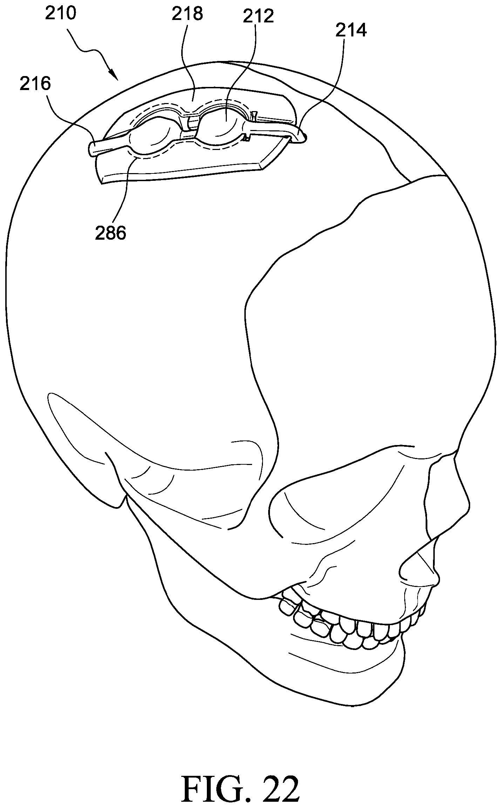

In accordance with the embodiment disclosed with reference to Figure 19 (not part of the present invention), physical bumps 280a-d are provided on the housing 218 of the shunt plug 210. While four bumps are shown in accordance with a disclosed embodiment, it is appreciated the number and location of the bumps may be varied to suit specific needs. In accordance with the embodiment disclosed with reference to Figure 20 (not part of the present invention), magnets or ferromagnetic properties 282 are integrated into the housing 218. The magnets or ferromagnetic properties 282 are oriented in the housing 218 to allow an external magnet to be employed in triangulating a particular location upon the shunt plug 210. In accordance with the embodiment disclosed with reference to Figure 21 (not part of the present invention), an RFID (radio-frequency identification) device 284 is embedded in the housing 218 of the shunt plug 210 with the ability to identify a point on the shunt plug 210. In accordance with the embodiment disclosed with reference to Figure 22, radiographic and/or acoustic properties 286 are integrated into the housing 218 that allow specific points of the shunt plug 210 to be seen by imaging modalities (CT, MRI, X-ray, Ultrasound, etc...). While the various identification devices described above are integrated into the housing, it is appreciated they might also be integrated into the shunt valve without departing from the spirit of the present invention.

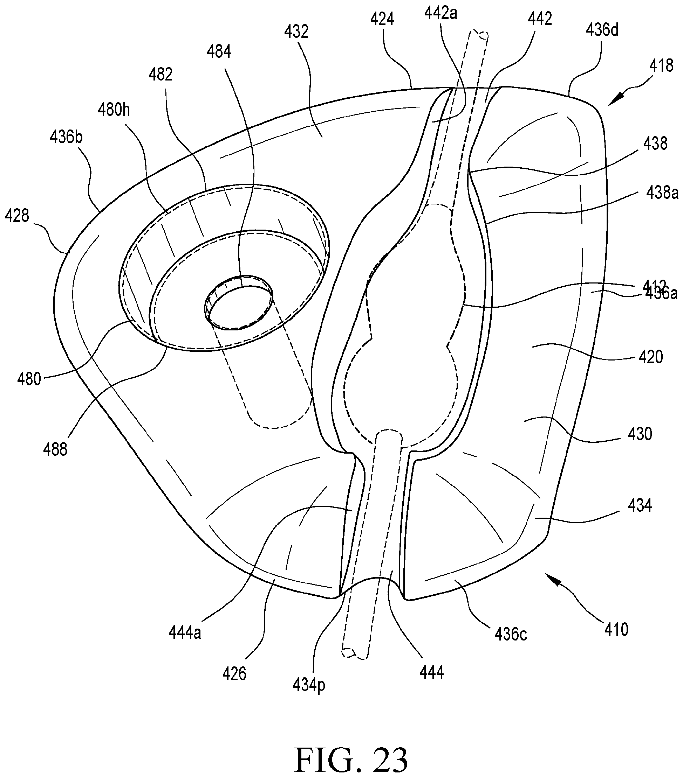

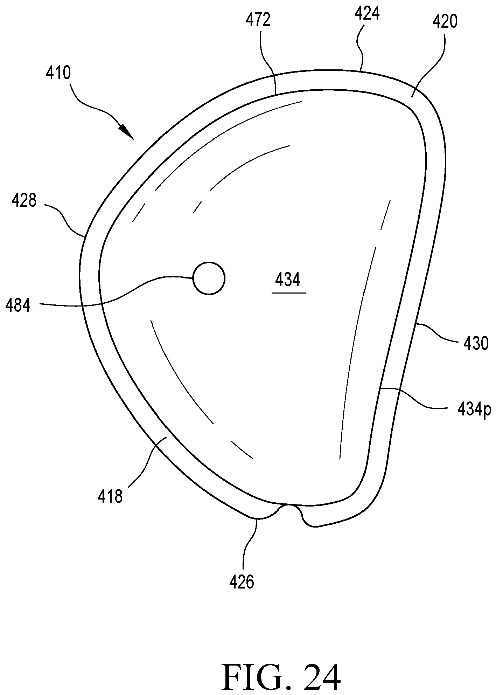

Referring to Figures 23 to 25, an embodiment of the present cerebral spinal fluid shunt plug 410 according to the invention is disclosed. As with the prior embodiments, the shunt plug 410 is shaped and dimensioned for positioning within a physician formed cranial hole 500 and is further shaped and dimensioned for housing a shunt valve 412 in a reliable and secure manner so that a ventricular catheter 414 and peritoneal catheter 416 may be positioned without fear that the shunt valve 412 might move and/or the catheters 414, 416 might become disengaged from their desired locations. Still further, this embodiment is shaped and dimensioned for integration of a wireless intracranial monitoring device 480 with the shunt plug 410.

The shunt plug 410 includes a shunt plug housing 418 composed of a bottom first housing member 420. In accordance with the disclosed embodiments, the shunt valve 412 and the wireless intracranial monitoring device 480 are placed within the shunt plug housing 418, so as to create the shunt plug 410 of the present invention, at the time of surgery.

The shunt plug housing 418 is substantially triangular shaped (with curved and extended corners, as well as arcuate walls) and includes a first end 424, a second end 426, a short first lateral side 428, and a long second lateral side 430. However, and as with the prior embodiments, it is appreciated various shapes may be employed within the spirit of the present invention and the shape of the shunt plug housing may be varied without departing from the spirit of the present invention.

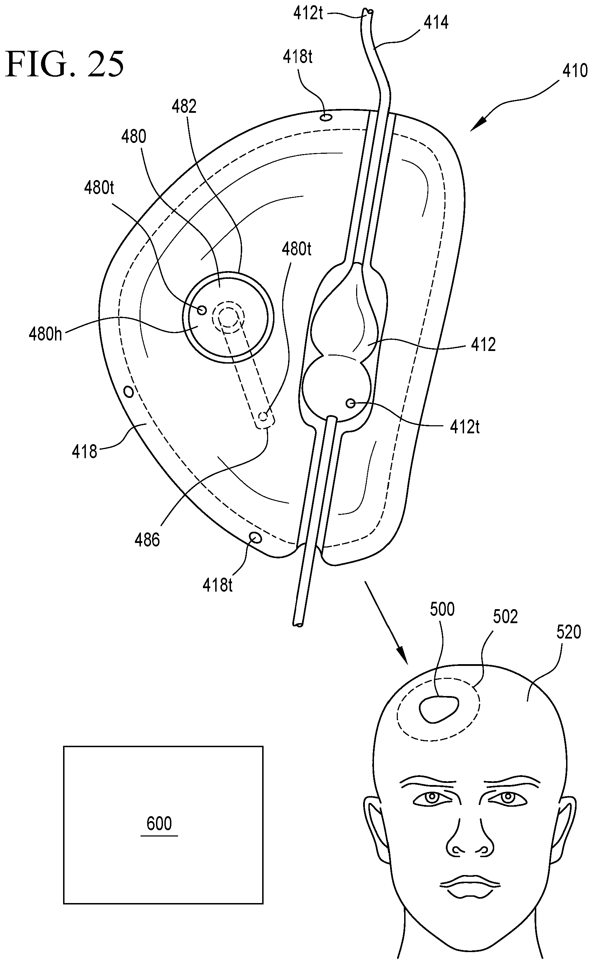

The shunt plug housing 418 also includes an upper surface 432, a lower surface 434, and continuous side walls 436a-d extending between the upper surface 432 and the lower surface 434, as well as about the periphery of the shunt plug housing 418. As will be appreciated based upon the following disclosure, and as with the embodiment of Figures 9-18, the lower surface 434 is provided with a projection 434p that ultimately fits within the cranial hole 500 to assist in holding the shunt plug 410 in position after installation. With this in mind, the projection 434p is shaped to fit within the cranial hole 500 as shown in Figure 25.

While a particular shape of the shunt plug housing 418 in accordance with the disclosed embodiment is disclosed herein for the purpose of explaining the present invention, it is appreciated various shapes may be employed within the spirit of the present invention. As such, the shape of the shunt plug and the mechanism for the creation of the cranial hole are intimately related and may be varied based upon various needs and requirements. For example, and in contrast with the embodiments described above with reference to Figures 1 to 22, the shunt plug housing includes a substantially triangular shape.

A shunt valve recess 438 is formed within the upper surface 432 of the shunt plug housing 418. The shunt valve recess 438 is in communication with the exterior of the shunt plug housing 418 via access passageways 442, 444 extending from the exterior surface of the shunt plug housing 418 to the shunt valve recess 438. As will be explained below in greater detail, these access holes (or passageways) 442, 444 allow for connection of the ventricular catheter 414 and the peritoneal catheter 416 with the shunt valve 412 housed within the shunt valve recess 438 of the shunt plug housing 418. The access passageways 442, 444 are defined by recessed surfaces formed along the upper surface 432 of the shunt plug housing 418. Depending upon the shape of the shunt plug housing 418 and the shunt valve 412 to be positioned therein, the position of the access holes (or passageways) 442, 444 may be varied to optimize the ultimate positioning of the peritoneal catheter 416 and the ventricular catheter 414.

As discussed above, the shunt valve recess 438 in which the shunt valve 412 is positioned, as well as the access holes 442, 444 for the passage of the ventricular and peritoneal catheters 414, 416, are formed within the shunt plug housing 418. The shunt valve recess 438 and access holes 442, 444 are defined by recessed surfaces 438a, 442a, 444a formed along the upper surface 432 of the shunt plug housing 418. In particular, the recessed surface 438a defining the shunt valve recess 438 is formed along the upper surface 432 of the shunt plug housing 418; the recessed surface 442a defining the first access hole (or passageway) 442 is formed along the upper surface 432 adjacent the first end 424; and the recessed surfaces 444a defining the second access hole (or passageway) 444 are formed along the side wall 464a of the shunt plug housing 418 at the second end 426 thereof.

As briefly discussed above, the shunt valve recess 438 defined within the shunt plug housing 418 is shaped and dimensioned for placement of the shunt valve 412 therein. As those skilled in the art will appreciate, and as explained above in conjunction with the prior embodiment, a variety of shunt valves are known in the art and the present shunt plug housing 418 may be adapted to accommodate a variety of these shunt valves. The present invention may also be used in conjunction with the Rickam reservoir and other similar reservoirs used in cerebral spinal fluid management. In accordance with a preferred embodiment, the shunt plug housing 418 should have a surface area along its upper surface 432 of at least five cm² so as to accommodate various shunt valves and to provide the necessary space for placement of the shunt valve 412 within the shunt valve recess 438 defined within the shunt plug housing 418.

As will be explained below in detail, once the shunt valve 412 is positioned within the shunt valve recess 438 of the shunt plug housing 418 the shunt plug 410 of the present invention may be utilized for the purpose of performing a cerebral spinal fluid shunt procedure.

In addition to the shunt valve recess 438 for the shunt valve 412 as discussed above, the shunt plug housing 418 of this embodiment further includes an intracranial monitoring device recess 482 formed within the upper surface 432 of the shunt plug housing 418 adjacent to the short first lateral side 428. The intracranial monitoring device recess 482 is shaped and dimensioned for positioning of an intracranial monitoring device 480, in particular, the head 480h of the intracranial monitoring device 480, therein. As such, and as will be appreciated based upon the following disclosure, the intracranial monitoring device recess 482 is provided with a central access hole 484 extending from the intracranial monitoring device recess 482 to the lower surface 434 of the shunt plug housing 418. The central access hole 484 is shaped and dimensioned for the passage of the probe 486 of the wireless intracranial monitoring device 480 therethrough and to a desired position within the brain.

The intracranial monitoring device recess 482 in which the wireless intracranial monitoring device 480 is positioned, as well as the central access hole 484 for the passage of the probe 486, is formed within the shunt plug housing 418. The intracranial monitoring device recess 482 is defined by recessed surfaces 488 formed along the upper surface 432 of the shunt plug housing 418. In particular, the recessed surface 488 defining the intracranial monitoring device recess 482 is formed along the upper surface 432 of the shunt plug housing 418.

As briefly discussed above, the intracranial monitoring device recess 482 defined within the shunt plug housing 418 is shaped and dimensioned for placement of the wireless intracranial monitoring device 480 therein. As those skilled in the art will appreciate, and as explained above in conjunction with the prior embodiment, a variety of wireless intracranial monitoring devices are known in the art and the present shunt plug housing 418 may be adapted to accommodate a variety of these wireless intracranial monitoring device 480. However, and in accordance with a preferred embodiment of the present invention, the wireless intracranial monitoring device 480 is one or a combination of the wireless intracranial pressure monitoring devices disclosed in U.S. Patent Nos. 8,337,413 and 9,339,189 and U.S. Patent Application Publication Nos. 2006/0025704, 2008/0161659, 2008/0262319, 2011/0009716, 2013/0123660, and 2014/0210637. In accordance with this embodiment, the shunt plug housing 418 should have a surface area along its upper surface 432 sufficient to accommodate various shunt valves and wireless intracranial monitoring devices.

The inclusion of the wireless intracranial monitoring device 480 with the shunt plug 410 of the present invention results in a reduction in the pressure generated by mounting implantable devices on the scalp and allows for measurement of the cerebral spinal fluid manage by the shunt valve 412 itself.

Further functionality may be achieved by using a wireless intracranial monitoring device offering multiple sensing capabilities (multimodal), for example, as disclosed in U.S. Patent Application Publication No. 2018/0325386, entitled "MULTIPLE IMPLANTABLE SENSOR PROBE," published November 15, 2018.

Once the shunt valve 412 and the wireless intracranial monitoring device 480 are positioned within the shunt valve recess 438 of the shunt plug housing 418 the shunt plug 410 of the present invention may be utilized for the purpose of performing a cerebral spinal fluid shunt procedure as explained above.

With the inclusion of a wireless intracranial monitoring device 480 with the shunt plug 410 of the present invention, positioning of the shunt plug 410 becomes critical. As such, the installation procedure is modified as described below.

Referring to Figure 25, the procedure is first initiated by making the required incision for passage of the peritoneal catheter 416. Thereafter, a cranial incision is made and the cranial hole 500 in the skull 520 is created utilizing a template 502 (shown in broken lines). In accordance with a preferred embodiment, and considering the triangular shape of the cranial hole 500, burr holes are formed at the respective ends of the template 502, and the remainder of the skull 520 is cut away along the lines as defined by the template 502. As with the prior embodiment, it is appreciated the cranial hole may be made using any method acceptable to those skilled in the art. Given the matching shape of the cranial hole 500 and the shunt plug 410, the shunt plug 410 will fit snugly within the cranial hole 500 thereby minimizing potential movement after completion of the procedure.

With the cranial hole 500 completed, the ventricular catheter 414 is positioned within the ventricle and the peritoneal catheter 416 is positioned with the body as using well know medical procedures. Thereafter, the shunt plug housing 418 is positioned within the cranial hole 500 with the upper surface 432 facing upwardly, and the ventricular catheter 414 is cut to an appropriate length. The ends of the peritoneal catheter 416 and the ventricular catheter 414 adjacent the shunt plug 410 are then secured to the shunt valve 412 and the shunt valve 412 is positioned within the shunt plug housing 418. In particular, the shunt plug 10 is mounted within the cranial hole 500 such that the upper surface 432 is substantially flush with the outer surface of the skull 520 and the projection 434p along the lower surface 434 is positioned within the cranial hole 500. As such, portions along the periphery of the shunt plug housing 418 overlie the skull 520, and screws may be passed therethrough to facilitate secure attachment of the shunt plug 410 to the skull. It is, however, appreciated the exact positioning of the shunt plug will vary based upon specific anatomical characteristics of the patient. Once the shunt plug 410 is properly positioned and secured in place using known techniques, the wireless intracranial monitoring device 480 is positioned within the intracranial monitoring device recess 482 with the probe 486 extending into the brain.

As those skilled in the art will appreciate, proper positioning of the probe 486 of the intracranial monitoring device 480 and the ventricular catheter 414 of the shunt valve 412 are critical. The orientation of the wireless intracranial monitoring device 480, for example, the probe 486 of the intracranial monitoring devices 480, relative to ventricular catheter 414 of the shunt valve 412 is critical to understand and appreciate so as to avoid eloquent structures on the cortex of the brain; such as the trajectory between Kocher's point and the ventricles or previously necrosed brain damage in a traumatic injury or due to high intracranial pressure. Furthermore, by identifying the relative positions of the wireless intracranial monitoring device 480 and the shunt valve 412 to the cortex, the relationship of the shunt valve 412 and wireless intracranial monitoring device 480 relative to a target within the brain is surmised and eloquent structures of the brain are avoided. For example, and through the use of the present shunt plug 410 in conjunction with various computer based surgical guidance systems 600 as discussed below, it is possible for surgeons to fully appreciate the relationship of the intracranial monitoring device 480, the shunt valve 412, and/or the ventricular catheter 418 in relation to the shunt plug 410 and, therefore, the cortex. This enables the surgeon to place the intracranial monitoring device 480, the shunt valve 412, and/or the ventricular catheter 418 in a manner that minimizes the potential for cortical damage.

In practice, and prior to initiating the surgical procedure, virtual images of the shunt plug 410, including both the shunt valve 412 and the intracranial monitoring device 480, are generated. Virtual images of the patient, including the approximate location of the shunt plug 410 are also generated. Upon initiation of the surgical procedure movement of the actual shunt plug 410, including the shunt valve 412, shunt plug housing 418, and the intracranial monitoring device 480, relative to the patient is monitored in real-time. This is achieved by the integration of tracking devices 412t, 418t, 480t into or onto the respective shunt valve 412, shunt plug housing 418, and the intracranial monitoring device 480. Additional tracking devices may be applied to the patient in a manner known to those skilled in the art. It should be appreciated that the tracking devices 412t, 418t, 480t may take a variety of forms so long as the computer-based guidance system 600 is capable of identifying the real-time movement of the various components of the shunt plug 410 being tracked. For example, the tracking devices may take the form of external tracking devices attached to the shunt plug, tracking devices integrated into the shunt plug, or existing structures of the shunt plug that are readily identifiable via the sensing structure of the computer-based guidance system 600. Sensing may be achieved via various known techniques, including, but not limited to, infrared, electromagnetic, optical, etc. sensing techniques.

With this information and using a computer based surgical guidance system 600, the shunt plug 410 is properly positioned within the patient. Once the shunt valve 412 and the wireless intracranial monitoring device 480 are properly positioned, they may be actuated utilizing well known procedures, and the procedure is completed in accordance with known medical procedures.

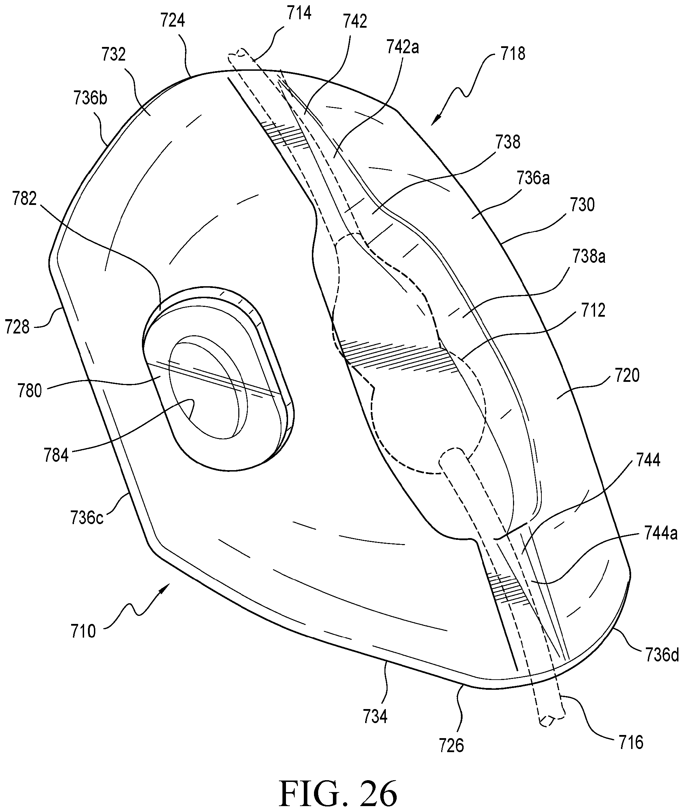

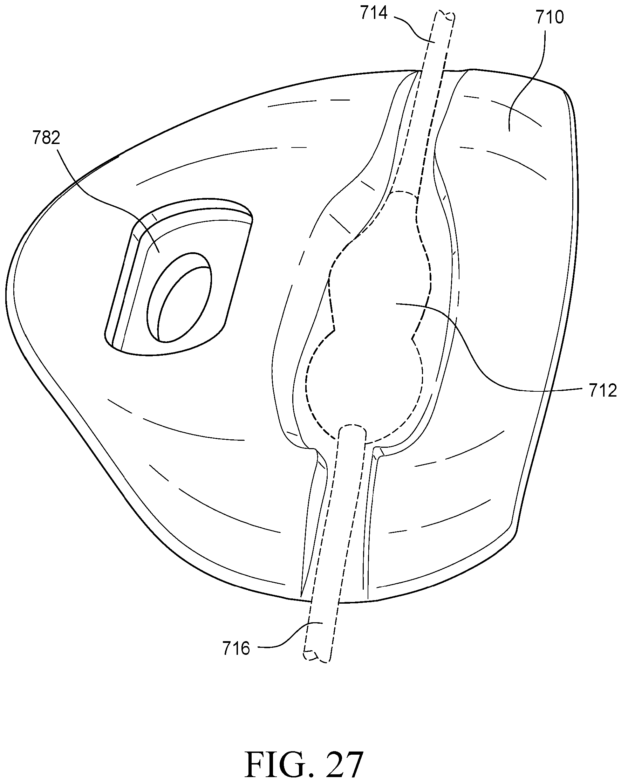

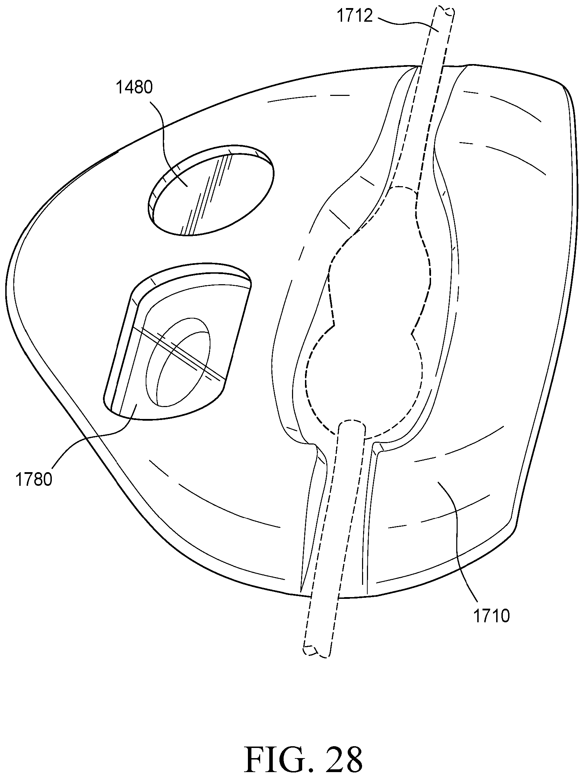

Referring to Figures 26 and 27, yet another embodiment of the present cerebral spinal fluid shunt plug 710 is disclosed. As with the prior embodiments, the shunt plug 710 is shaped and dimensioned for positioning within a physician formed cranial hole and is further shaped and dimensioned for housing a shunt valve 712 in a reliable and secure manner so that a ventricular catheter 714 and peritoneal catheter 716 may be positioned without fear that the shunt valve 712 might move and/or the catheters 714, 716 might become disengaged from their desired locations. Still further, this embodiment is shaped and dimensioned for integration of a window in the form of a lucent disk 780 with the shunt plug 710. Given the similarity between the embodiment disclosed with reference to Figures 23 to 25 and the embodiment disclosed with reference to Figures 26 and 27, it is appreciated one shunt plug housing 718 may be used in conjunction with either the wireless intracranial monitoring device 480 of the embodiment disclosed with reference to Figures 23 to 25 or the lucent disk 780 of the embodiment disclosed with reference to Figures 26 and 27. Further still, and with reference to Figure 28, an embodiment combining the lucent disk 1780 and the wireless intracranial monitoring device 1480 for use with a shunt valve 1712 is disclosed.

The shunt plug 710 includes a shunt plug housing 718 composed of a bottom first housing member 720. In accordance with the disclosed embodiments, the shunt valve 712 and the lucent disk 780 are placed within the shunt plug housing 718, so as to create the shunt plug 710 of the present invention, at the time of surgery.

The shunt plug housing 718 is substantially triangular shaped (with curved and extended corners, as well as arcuate walls) and includes a first end 724, a second end 726, a short first lateral side 728, and a long second lateral side 730. However, and as with the prior embodiments, it is appreciated various shapes may be employed within the spirit of the present invention and the shape of the shunt plug housing may be varied without departing from the spirit of the present invention.

The shunt plug housing 718 also includes an upper surface 732, a lower surface 734, and continuous side walls 736a-d extending between the upper surface 732 and the lower surface 734, as well as about the periphery of the shunt plug housing 718. As will be appreciated based upon the following disclosure, and as with the embodiment of Figures 9-18, the lower surface (not shown) is provided with a projection (not shown) that ultimately fits within the cranial hole to assist in holding the shunt plug 710 in position after installation. With this in mind, the projection is shaped to fit within the cranial hole 500 as shown in Figure 25.

While a particular shape of the shunt plug housing 718 in accordance with the disclosed embodiment is disclosed herein for the purpose of explaining the present invention, it is appreciated various shapes may be employed within the spirit of the present invention. As such, the shape of the shunt plug and the mechanism for the creation of the cranial hole are intimately related and may be varied based upon various needs and requirements. For example, and in contrast with the embodiments described above with reference to Figures 1 to 22, the shunt plug housing includes a substantially triangular shape.

A shunt valve recess 738 is formed within the upper surface 732 of the shunt plug housing 718. The shunt valve recess 738 is in communication with the exterior of the shunt plug housing 718 via access passageways 742, 744 extending from the exterior surface of the shunt plug housing 718 to the shunt valve recess 738. As will be explained below in greater detail, these access holes (or passageways) 742, 744 allow for connection of the ventricular catheter 714 and the peritoneal catheter 716 with the shunt valve 712 housed within the shunt valve recess 738 of the shunt plug housing 718. The access passageways 742, 744 are defined by recessed surfaces formed along the upper surface 732 of the shunt plug housing 718. Depending upon the shape of the shunt plug housing 718 and the shunt valve 712 to be positioned therein, the position of the access holes (or passageways) 742, 744 may be varied to optimize the ultimate positioning of the peritoneal catheter 716 and the ventricular catheter 714.

As discussed above, the shunt valve recess 738 in which the shunt valve 712 is positioned, as well as the access holes 742, 744 for the passage of the ventricular and peritoneal catheters 714, 716, are formed within the shunt plug housing 718. The shunt valve recess 738 and access holes 742, 744 are defined by recessed surfaces 738a, 742a, 744a formed along the upper surface 732 of the shunt plug housing 718. In particular, the recessed surface 738a defining the shunt valve recess 738 is formed along the upper surface 732 of the shunt plug housing 718; the recessed surface 742a defining the first access hole (or passageway) 742 is formed along the upper surface 732 adjacent the first end 724; and the recessed surfaces 744a defining the second access hole (or passageway) 744 are formed along the side wall 736a of the shunt plug housing 718 at the second end 726 thereof.

As briefly discussed above, the shunt valve recess 738 defined within the shunt plug housing 718 is shaped and dimensioned for placement of the shunt valve 712 therein. As those skilled in the art will appreciate, and as explained above in conjunction with the prior embodiment, a variety of shunt valves are known in the art and the present shunt plug housing 718 may be adapted to accommodate a variety of these shunt valves. The present invention may also be used in conjunction with the Rickam reservoir and other similar reservoirs used in cerebral spinal fluid management. In accordance with a preferred embodiment, the shunt plug housing 718 should have a surface area along its upper surface 732 of at least five cm² so as to accommodate various shunt valves and to provide the necessary space for placement of the shunt valve 712 within the shunt valve recess 738 defined within the shunt plug housing 718.

As will be explained below in detail, once the shunt valve 712 is positioned within the shunt valve recess 738 of the shunt plug housing 718 the shunt plug 710 of the present invention may be utilized for the purpose of performing a cerebral spinal fluid shunt procedure.

In addition to the shunt valve recess 738 for the shunt valve 712 as discussed above, the shunt plug housing 718 of this embodiment further includes a window recess 782 formed within the upper surface 732 of the shunt plug housing 718 adjacent to the short first lateral side 728. The window recess 782 is shaped and dimensioned for positioning of a lucent disk 780. As such, and as will be appreciated based upon the following disclosure, the window recess 782 is provided with a central access hole 784 extending from the window recess 782 to the lower surface 734 of the shunt plug housing 718. The central access hole 784 is shaped and dimensioned for the passage of light, sound, and/or radio waves therethrough so as to access the brain for imaging and treatment.

The window recess 782 in which the lucent disk 780 is positioned, as well as the central access hole 784, is formed within the shunt plug housing 718. The window recess 782 is defined by recessed surfaces 788 formed along the upper surface 732 of the shunt plug housing 718. In particular, the recessed surface 788 defining the window recess 782 is formed along the upper surface 732 of the shunt plug housing 718.

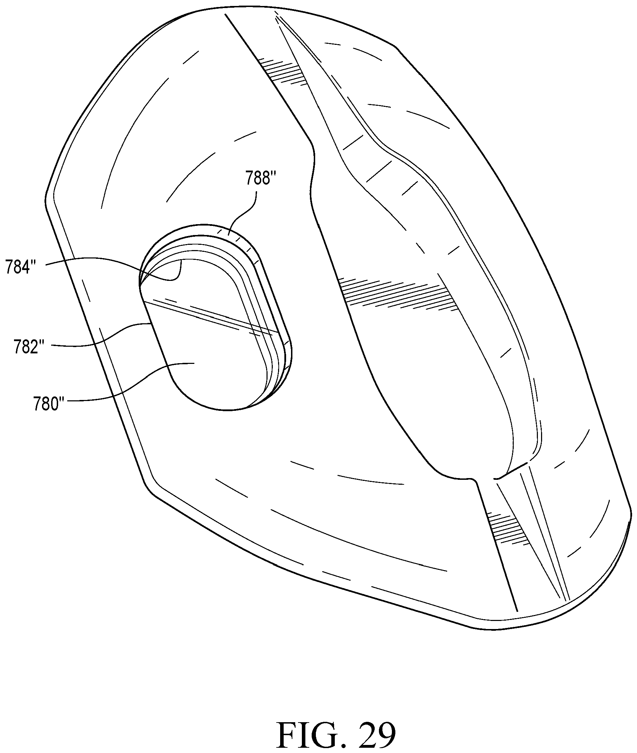

While the embodiment described considers a situation wherein the shunt plug housing 718 may be used in conjunction with either the wireless intracranial monitoring device 480 of the embodiment disclosed with reference to Figures 23 to 25 or the lucent disk 780 of the embodiment disclosed with reference to Figures 26 and 27, the shunt plug housing may be specifically designed for use with only the lucent disk. Considering such an embodiment, and with reference to Figure 29, the central access hole 784" would be substantially enlarged such that the recessed surface 788" positioned about the central access hole 784" is made relatively small and is constructed to function as a ledge supporting the bottom surface of the lucent disk 780" when it is positioned within the window recess 782". By expanding the central access hole 784", unattenuated passage of light, sound, radio, and other waves will be optimized.

As briefly discussed above, the window recess 782 defined within the shunt plug housing 718 is shaped and dimensioned for placement of the lucent disk 780 therein. In accordance with a preferred embodiment of the present invention, the lucent disk 780 is optically transparent, optically translucent to all light waves, sonolucent (that is, allowing passage of ultrasonic waves without production of echoes that are due to reflection of some of the waves), and/or radiolucent (that is, allowing passage of radio waves without production of echoes that are due to reflection of some of the waves). Further still, and in accordance with a disclosed embodiment, the lucent disk is made of polymethyl methacrylate (PMMA).

Through the provision of a lucent disk, a variety of options are available to medical practitioners wishing to provide the best treatment options to their patients. For example, lucent disk may be manufactured in a manner allowing for the transmission of ultrasonic waves as described in U.S. Patent No. 9,044,195, entitled "IMPLANTABLE SONIC WINDOW," ('195 Patent). As explained in the '195 Patent, a strong, porous sonically translucent material through which ultrasonic waves can pass for purposes of imaging the brain is employed, wherein the material is a polymeric material, such as polyethylene, polystyrene, acrylic, or poly(methyl methacrylate) (PMMA). In addition, U.S. Patent No. 9,535,192, entitled "METHOD OF MAKING WAVEGUIDE-LIKE STRUCTURES," ('192 Publication) and U.S. Patent Application Publication No. 2017/0156596, entitled "CRANIAL IMPLANTS FOR LASER IMAGING AND THERAPY," ('596 Publication) making waveguide-like structures within optically transparent materials using femtosecond laser pulses wherein the optically transparent materials are expressly used in the manufacture of cranial implants. The '596 publication explains the use of optically transparent cranial implants and procedures using the implants for the delivery of laser light into shallow and/or deep brain tissue. The administration of the laser light can be used on demand, thus allowing real-time and highly precise visualization and treatment of various pathologies. Further still, Tobias et al. describe an ultrasound window to perform scanned, focused ultrasound hyperthermia treatments of brain tumors. Tobias et al., "ULTRASOUND WINDOW TO PERFORM SCANNED, FOCUSED ULTRASOUND HYPERTHERMIA TREATMENTS OF BRAIN TUMORS," Med. Phys. 14(2), Mar/Apr 1987, 228-234. Tobias et al. tested various materials to determine which material would best serve as an acoustical window in the skull and ultimately determined polyethylene transmitted a larger percentage of power than other plastics and would likely function well as an ultrasonic window. Further still, Fuller et al., "REAL TIME IMAGING WITH THE SONIC WINDOW: A POCKET-SIZED, C-SCAN, MEDICAL ULTRASOUND DEVICE," IEEE International Ultrasonics Symposium Proceedings, 2009, 196-199 provides further information regarding sonic windows.

Radiolucency as applied to the present invention allows a clinician to see the anatomy beneath the lucent disk 780 without "scatter" or interfering artifacts from the implant for diagnosis and follow-up. By another definition of radiolucency, radio waves are able to transmit easily through the lucent disk 780, for example, via Bluetooth or other frequency transmission; which can serve many purposes including, but not limited to, data management and controller telemetry. The provision of radiolucency also allows for the integration of markings (as discussed below) made with radiographic materials, for example, barium sulfate, to be visible in contrast to the remainder of the craniofacial implant to allow for unique device identifiers or unique patient information to be visible on post-operative scans.

Considering the provision of optical lucency in the lucent disk 780, the ability to optically transmit through the lucent disk 780 allows for: visualization of anatomy distal to the lucent disk 780, the potential of higher bandwidth optical links (similar to radio transmission) between proximal adjunct devices, light to be emitted through the lucent disk 780 to adjacent anatomy which could aid in optogenetics, and imaging/therapeutic modalities that rely on light like optical coherence tomography from within the implant. Of note, this was shown to be true on a postoperative (day 5) cranioplasty patient with the clear implant. Belzberg M, Ben Shalom N, Yuhanna E, Manbachi A, Tekes A, Huang J, Brem H, Gordon C, "Sonolucent Cranial Implants: Cadaveric study and Clinical Findings Supporting Diagnostic and Therapeutic Trans-Cranioplasty Ultrasound," J Craniofac Surg. (July/August 2019 - Volume 30 - Issue 5 - p 1456-1461).

With the inclusion of a lucent disk 780 with the shunt plug 710 of the present invention, positioning of the shunt plug 710 becomes critical. As such, the installation procedure is as described below.

The procedure is first initiated by making the required incision for passage of the peritoneal catheter 716. Thereafter, a cranial incision is made and the cranial hole in the skull is created utilizing a template. In accordance with a preferred embodiment, and considering the triangular shape of the cranial hole, burr holes are formed at the respective ends of the template, and the remainder of the skull is cut away along the lines as defined by the template. As with the prior embodiment, it is appreciated the cranial hole may be made using any method acceptable to those skilled in the art. Given the matching shape of the cranial hole and the shunt plug 710, the shunt plug 710 will fit snugly within the cranial hole thereby minimizing potential movement after completion of the procedure.

With the cranial hole completed, the ventricular catheter 714 is positioned within the ventricle and the peritoneal catheter 716 is positioned within the body using well know medical procedures. Thereafter, the shunt plug housing 718 is positioned within the cranial hole with the upper surface 732 facing upwardly, and the ventricular catheter 714 is cut to an appropriate length. The ends of the peritoneal catheter 716 and the ventricular catheter 714 adjacent the shunt plug 710 are then secured to the shunt valve 712 and the shunt valve 712 is positioned within the shunt plug housing 718. In particular, the shunt plug 710 is mounted within the cranial hole 500 such that the upper surface 732 is substantially flush with the outer surface of the skull 520 and the projection 734p along the lower surface 734 is positioned within the cranial hole. As such, portions along the periphery of the shunt plug housing 718 overlie the skull, and screws may be passed therethrough to facilitate secure attachment of the shunt plug 710 to the skull. It is, however, appreciated the exact positioning of the shunt plug will vary based upon specific anatomical characteristics of the patient. Once the shunt plug 710 is properly positioned and secured in place using known techniques, the lucent disk 780 is positioned within the window recess 782.

In practice, and prior to initiating the surgical procedure, virtual images of the shunt plug 710, including both the shunt valve 712 and the lucent disk 780, are generated. Virtual images of the patient, including the approximate location of the shunt plug 710 are also generated. Upon initiation of the surgical procedure, movement of the actual shunt plug 710, including the shunt valve 712, shunt plug housing 718, and the lucent disk 780, relative to the patient is monitored in real-time. This is achieved by the integration of tracking devices (as discussed above with the prior embodiment shown with reference to Figure 25) into or onto the respective shunt valve 712, shunt plug housing 718, and the lucent disk 780. Additional tracking devices may be applied to the patient in a manner known to those skilled in the art. It should be appreciated that the tracking devices may take a variety of forms so long as the computer-based guidance system is capable of identifying the real-time movement of the various components of the shunt plug 710 being tracked. For example, the tracking devices may take the form of external tracking devices attached to the shunt plug, tracking devices integrated into the shunt plug, or existing structures of the shunt plug that are readily identifiable via the sensing structure of the computer-based guidance system. Sensing may be achieved via various known techniques, including, but not limited to, infrared, electromagnetic, optical, etc. sensing techniques.

With this information and using a computer based surgical guidance system, the shunt plug 710 is properly positioned within the patient. Once the shunt valve 712 and the lucent disk 780 are properly positioned, they may be actuated utilizing well known procedures, and the procedure is completed in accordance with known medical procedures.

Considering the fact the lucent disk is optically transparent, optically translucent to all light waves, sonolucent, and/or radiolucent, various features have been integrated into the lucent disk in an effort to enhance the functionality thereof. While these features are described herein as individual embodiments, it is appreciated they may be combined in various combinations as the needs of a patient dictate.

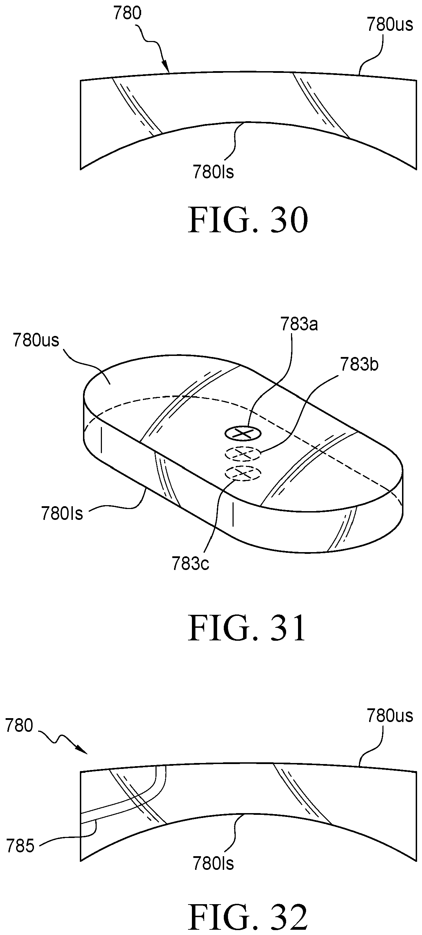

In accordance with one embodiment as shown with reference to Figure 30, the lucent disk 780' may be constructed with variations in shape designed to control the manner in which light, sound, radio, and other waves pass therethrough. Such variations in shape would be undertaken in a manner similar to the way in which eyeglasses are adjusted for each patient. For example, and with reference to the disclosed embodiment, the curvature of the upper surface 780us differs from the curvature of the lower surface 780ls wherein the upper surface 780us has a much larger radius of curvature.

In accordance with another embodiment as shown with reference to Figure 31, the lucent disk 780" may be constructed with an alignment feature 781. In accordance with a disclosed embodiment, the alignment feature 781 includes a series of markings 783a-c at different depths within the lucent disk. For example, an outer first lucent disk marking 783a and an inner second lucent disk marking 783b are formed along the upper and lower surfaces 780us, 780ls, respectively, of the lucent disk 780. One or more additional interior lucent disk markings 780c may be formed within the body of the lucent disk 780 and in alignment with the outer first lucent disk marking 783a and an inner second lucent disk marking 783b. While an outer first lucent disk marking 783a, an inner second lucent disk marking 783b, and at least one additional interior lucent disk marking 783c are disclosed herein, it is appreciated various combinations of markings may be used within the spirit of the present invention.

The outer first lucent disk marking 783a, the inner second lucent disk marking 783b, and the plurality of additional interior lucent disk markings 783c are aligned such that when a transmitter of light, sound, radio, or other waves is properly aligned with the markings, the light, sound, radio, or other waves will be directed to the proper location within the cranium. Similarly, when one looks through the lucent disk 780 and the outer first lucent disk marking 783a, the inner second lucent disk marking 783b, and the at least one additional interior lucent disk markings 783c merge into a single location identifying image (for example, crosshairs or circles), a specific brain anatomy (or other structural element upon the surface of the brain) is identified by the single location identifying image. When the specific brain anatomy identified by the single location identifying image changes over time, the surgeon will know that something has shifted and will take appropriate action.

In accordance with one embodiment as shown with reference to Figure 32, the lucent disk may be constructed with wire channels 785 oriented for various purposes specific to different patients and treatment protocols.

The use of a lucent disk offers various advantages to address specific needs within the industry. For example, patients requiring ventriculoperitoneal shunting are postoperatively left with a high-profile shunt valve underneath their scalp and have high rates of complication and revision due to infection, occlusion, extrusion, and/or migration (dislodging/disconnecting) of the valve. Additionally, monitoring of intracranial pressure and ventricular size can be difficult and costly when complications arise, requiring lumbar punctures and CT scans to diagnose effectively. Patients receiving and using the present lucent disk would benefit from reduced profile of the shunt valve (level with the skull), reducing complications associated with pressure on the valve and restoring the native contour of the cranium. As well, due to high level of complication involved with ventriculoperitoneal shunting, ultrasound diagnostics would prove beneficial to rapidly, and noninvasively, monitor the size of the ventricles to correlate efficacy of the shunt and potential necessity of revision.

In accordance with yet another embodiment, the lucent element is integrated with the spinal fluid shunt plug 210 as a selectively attachable accessory in the form of a clear custom intercranial implant.

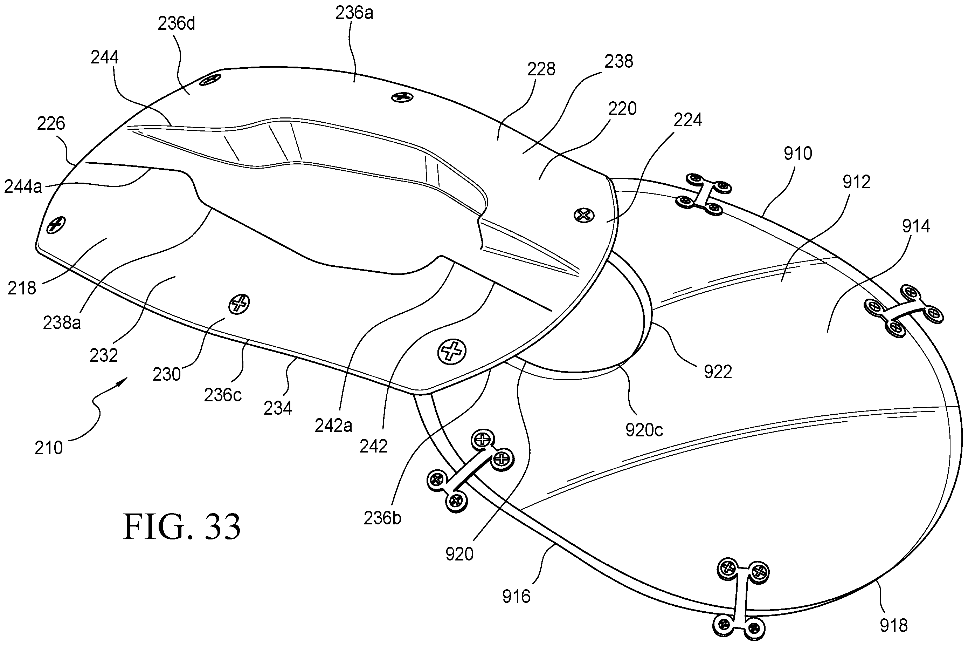

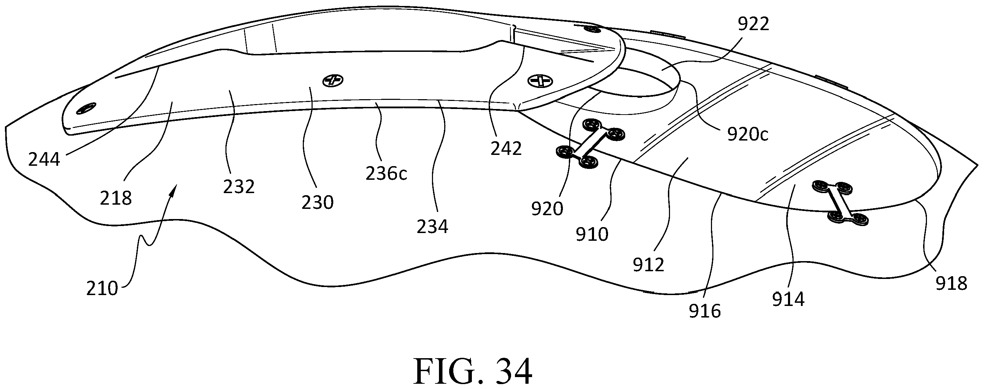

Referring to Figures 33 and 34, the spinal shunt plug is as described above with reference to Figures 9 to 18 and similar reference numerals will be used. The shunt plug 210 includes a shunt plug housing 218 composed of a bottom first housing member 220. The shunt plug housing 218 is substantially elliptically shaped (with curved and extended corners, as well as arcuate walls) and includes a first end 224, a second end 226, and first and second lateral sides 228, 230. However, and as with the prior embodiments, it is appreciated various shapes may be employed within the spirit of the present invention and the shape of the shunt plug housing may be varied without departing from the spirit of the present invention.

The shunt plug housing 218 also includes an upper surface 232, a lower surface 234, and continuous side walls 236a-d extending between the upper surface 232 and the lower surface 234, as well as about the periphery of the shunt plug housing 218. As will be appreciated based upon the following disclosure, and as with the embodiment of Figures 9-18, the lower surface 234 is provided with a projection (not shown) that ultimately fits within the cranial hole 1000 to assist in holding the shunt plug 210 in position after installation. With this in mind, the projection is shaped to fit within the cranial hole 500 as shown in Figure 25.

A shunt valve recess 238 is formed within the upper surface 232 of the shunt plug housing 218. The shunt valve recess 238 is in communication with the exterior of the shunt plug housing 218 via access passageways 242, 244 extending from the exterior surface of the shunt plug housing 218 to the shunt valve recess 238. As will be explained below in greater detail, these access holes (or passageways) 242, 244 allow for connection of the ventricular catheter 214 and the peritoneal catheter 216 with the shunt valve 212 housed within the shunt valve recess 238 of the shunt plug housing 218. The access passageways 242, 244 are defined by recessed surfaces formed along the upper surface 232 of the shunt plug housing 218. Depending upon the shape of the shunt plug housing 218 and the shunt valve 212 to be positioned therein, the position of the access holes (or passageways) 242, 244 may be varied to optimize the ultimate positioning of the peritoneal catheter 216 and the ventricular catheter 214.

As discussed above, the shunt valve recess 238 in which the shunt valve 212 is positioned, as well as the access holes 242, 244 for the passage of the ventricular and peritoneal catheters 214, 216, are formed within the shunt plug housing 218. The shunt valve recess 238 and access holes 242, 244 are defined by recessed surfaces 238a, 242a, 244a formed along the upper surface 232 of the shunt plug housing 218. In particular, the recessed surface 238a defining the shunt valve recess 238 is formed along the upper surface 232 of the shunt plug housing 218; the recessed surface 242a defining the first access hole (or passageway) 242 is formed along the upper surface 232 adjacent the first end 224; and the recessed surfaces 244a defining the second access hole (or passageway) 244 are formed along the side wall 264a of the shunt plug housing 218 at the second end 226 thereof.

As briefly discussed above, the shunt valve recess 238 defined within the shunt plug housing 218 is shaped and dimensioned for placement of the shunt valve 212 therein. As those skilled in the art will appreciate, and as explained above in conjunction with the prior embodiment, a variety of shunt valves are known in the art and the present shunt plug housing 218 may be adapted to accommodate a variety of these shunt valves.

As will be explained below in detail, once the shunt valve 212 is positioned within the shunt valve recess 238 of the shunt plug housing 218 the shunt plug 210 of the present invention may be utilized for the purpose of performing a cerebral spinal fluid shunt procedure.

The lucent element of this embodiment is a clear custom intercranial implant 910 shaped and dimensioned for positioning adjacent to the shunt plug 210. The clear custom intercranial implant 910 includes an implant body 912 structured in a manner as used and known by those skilled in the art of cranial surgical procedures. The implant body 912 may take a variety of forms and is most commonly shaped and dimensioned for integration into the structure of a patient's skull; that is, the implant body has a geometry that substantially conforms to a resected portion of the patient's anatomy to which the implant is to be secured. Briefly, the implant body 912 includes an outer (commonly convex) first surface 914, an inner (commonly concave) second surface 916, and a peripheral edge 918 extending between the outer first surface 914 and the inner second surface 916. The implant body 912 is shaped and dimensioned for engagement with the skull of the patient upon implantation in a manner well known to those skilled in the field of neurosurgical and neuroplastic reconstructive procedures. The outer first surface 914 and inner second surface 916 of the implant body 912 are preferably curved in a superior to inferior direction, a posterior to anterior direction, and a medial to lateral direction. In addition, the peripheral edge 918 has a substantial taper for resting upon a matching taper formed along the skull. It is, however, appreciated that this taper may vary (or not exist at all, that is, the peripheral edge may be substantially perpendicular relative to the outer first surface and the inner second surface) depending upon the specific needs.

In accordance with an embodiment, the implant body 912 is fabricated from a wide array of commonly-available biomaterials including, but not limited to, clear PMMA (Poly(methyl methacrylate)), PEEK (Polyether ether ketone), PEKK (Polyetherketoneketone), porous polyethylene, flexible silicone, cubic zirconium, titanium alloy, allograft, autograft, xenograft, glass, and/or various other tissue-engineered constructs. In fact, some of the biomaterials used in this novel device may be resorbable versus permanent with respect to time. In accordance with one embodiment, the implant body is ideally made of clear PMMA since it's fully translucent to light, sonolucent to ultrasound such that it allows for the passage of ultrasonic waves without the production of echoes that are due to reflection (as first described by Gordon et al. in "Sonolucent Cranial Implants: Cadaveric study and Clinical Findings Supporting Diagnostic and Therapeutic Trans-Cranioplasty Ultrasound. J Craniofac Surg 2019 and Gordon et al. in "Trans-Cranioplasty Ultrasound (TCU) through a Sonolucent Cranial Implant Made of Polymethyl methacrylate (PMMA): Phantom Study Comparing Ultrasound, CT and MRI. J Craniofac Surg 2019), permeable to low-coherence light used in optical coherence tomography (OCT), radiolucent such that it is permeable to various forms of electromagnetic radiation (for example, is permeable to ECoG (electrocorticographic) signals), and transparent for ideal visualization necessary for brain lead placement, catheter positioning, etc. This allows for the critical transmission of vital imaging with minimal distortion, such as direct visual inspection of the brain, ultrasound waves for brain pathology detection, low-coherence light used in optical coherence tomography (OCT), and wireless signal communication (i.e., electroencephalography or ECoG), which is essential for various neuromodulation devices. Another clear material that may be readily used in accordance with the present reconstructive cranial implant is cubic zirconium or plastic.

The optical clarity of the implant body 912 is important in that it provides for the provision of high optical clarity allowing for optical links connecting the external environment to the surface of the brain (for example, transmitting between the cortex and the other side of the reconstructive cranial implant). Visualization devices such as high-definition cameras, ultrasound probes, or optical coherence tomography (OCT) imaging devices may therefore be used in conjunction with the reconstructive cranial implant.

Still further, the implant body is constructed of a material allowing for imaging of the brain through the reconstructive cranial implant, for example, via ultra-sound or optical coherence tomography. It is known that clear PMMA will provide the ability to permit ultra-sound imaging of the brain therethrough so long as it is manufactured without additives that might function to block the radio waves of the imaging device.

While the majority of the peripheral edge 918 of the implant body defines a generally continuously curved surface shaped and dimensioned to sit within the resected portion of the skull, a mating segment 920 of the peripheral edge 918 of the implant body 912 is shaped and dimensioned for a mating coupling with the shunt plug housing 218.

In particular, and considering the curved shape of the shunt plug housing 218 at the first end 242 thereof, the mating segment 920 is formed with a relatively concave profile shaped and dimensioned to mate with the first end 242 of the shunt plug housing 218. At a central portion 920c of the mating segment, a further concave cut-out 922 is formed. The concave cut-out 922 is positioned for alignment with the first access passageways 242 and provides an opening for the ventricular catheter 214 for positioning a described above.

In practice, installation would be accomplished in a manner similar to the embodiments discussed above.

As with the embodiment disclosed above, various features may be integrated into the clear custom cranial implant in an effort to enhance the functionality thereof. While these features are described herein as individual embodiments, it is appreciated they may be combined in various combinations as the needs of a patient dictate.

While the shunt plug housing and implant body of the various embodiments disclosed above are made of various materials as discussed above, it is contemplated, the shunt plug housing and/or implant body implant body could be of a multi-material construction with the use of different materials in different elements of the shunt plug housing and/or implant body so as to expand the functionality thereof.

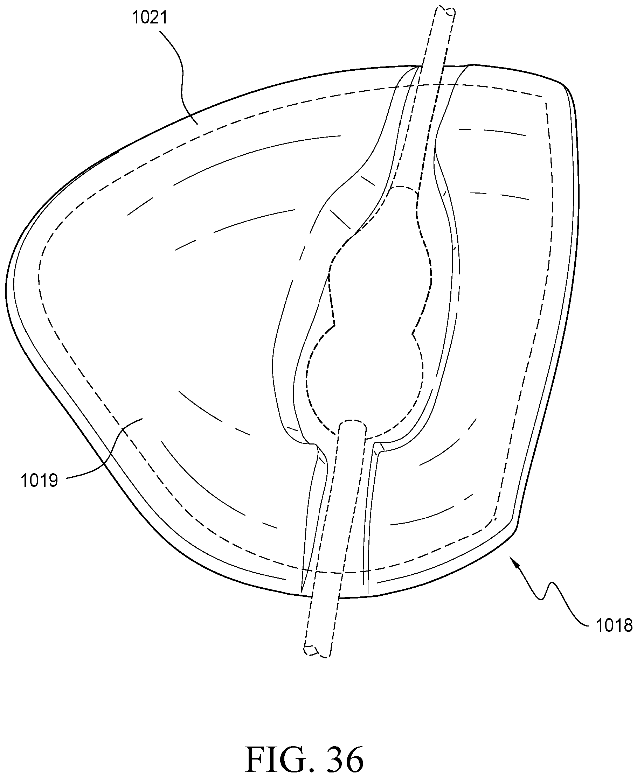

For example, and with reference to Figures 36 to 38 (not part of the present invention), the shunt plug housing 1018 includes a central body member 1019 made of rigid sonolucent PMMA and a flexible perimeter member 1021 made of porous polyethylene. Such a construction provides medical practitioners with a large sonolucent area for transcranioplasty ultrasound as provided by the central body member 1019 and a malleable perimeter as provided by the perimeter member 1021 that optimizes a smooth transition between the implant perimeter and the native skull.

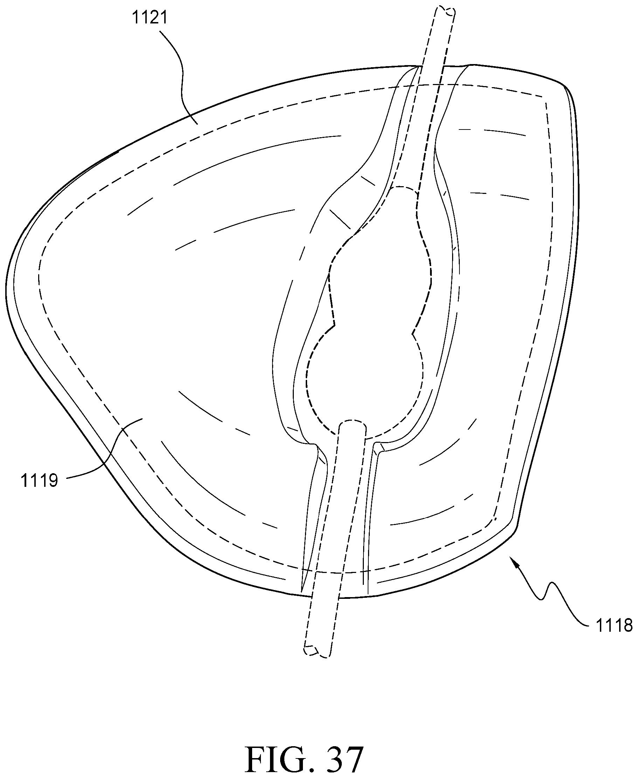

In accordance with another embodiment as shown with reference to Figure 37, the shunt plug housing 1118 includes a central body member 1119 made of cubic zirconium (or any other rigid sonolucent material) and a flexible perimeter member 1021 made of expanded polytetrafluoroethylene (EPTFE), silicon, or other malleable material.

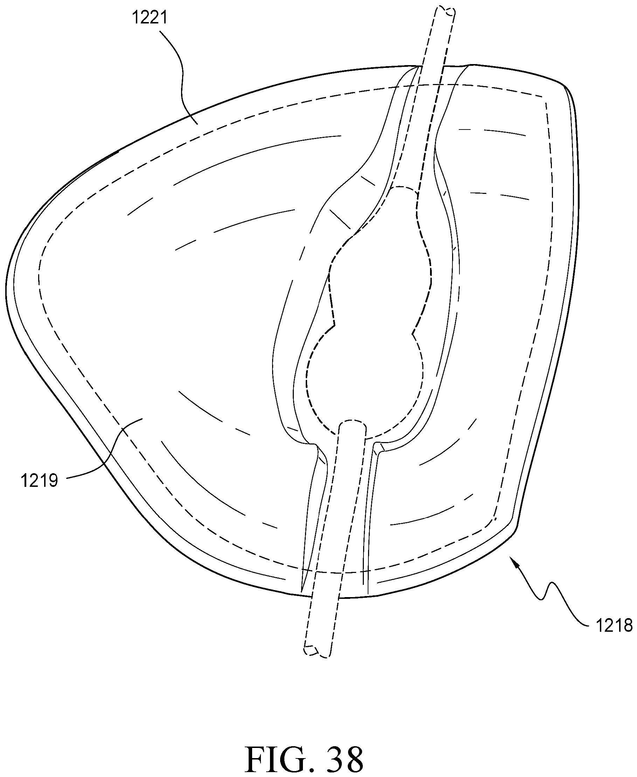

In accordance with yet another embodiment as shown in Figure 38 (not part of the present invention), the shunt plug housing 1218 could be a different composition of the same material, with the different compositions being selected to enhance sonolucency and aesthetic fixation. For example, the central body member 1219 of the shunt plug housing 1218 could be rigid sonolucent PMMA while the flexible perimeter member 1221 of the shunt plug housing 1218 could be PMMA with elastomer additives that change the material properties of the shunt plug housing from rigid to malleable. **In** this way, the implant could have an optimal smooth transition from the perimeter of the implant to the native skull.

While the embodiments presented above discuss the multi-material possibilities with regard to shunt plug housings only used with a shunt valve, such multi-material constructions could also be applied to the construction of implant bodies as disclosed with the other embodiments disclosed herein.

## Claims

1. A cerebral spinal fluid shunt plug (410, 710), comprising:
a shunt plug housing (418, 718) including an upper surface (432, 732), a lower surface (434), continuous side walls extending between the upper surface (432, 732) and the lower surface (434), as well as about the periphery of the shunt plug housing, a shunt valve recess (438, 738) being formed in the upper surface (432, 732) of the shunt plug housing (418, 718) and either an intracranial monitoring recess (482) with an access hole (484) or a window recess (782) with a central access hole (784) being formed in the upper surface (432, 732) of the shunt plug housing (418, 718),
the shunt plug housing (418, 718) further including passageways allowing the shunt valve recess (438, 738) to communicate with an exterior of the shunt plug housing, the access holes (484, 784) or passageways being shaped and dimensioned to allow for connection of a ventricular catheter and a peritoneal catheter with the shunt valve housed within the recess (438, 738) of the shunt plug housing (418, 718), the housing (418, 718) being shaped and dimensioned for positioning within a physician formed cranial hole (500) in a manner allowing the upper surface (432, 732) of the housing (418, 718) to be substantially flush with an outer surface of a skull and a projection (434p, 734p) along the lower surface being positioned within the cranial hole; and
a window or an intracranial monitoring device (480) shaped and dimensioned for respectively positioning within either the window recess (782) or the intracranial monitoring recess (482) of the shunt plug housing (418, 718).

2. The cerebral spinal fluid shunt plug (410, 710) according to claim 1, wherein the recess with an access hole is a window recess (782) with an access hole (784) and the cerebral spinal fluid shunt plug (410, 710) includes the window shaped and dimensioned for positioning within the window recess (782), the cerebral spinal fluid shunt plug (410, 710) further including an intracranial monitoring device recess (482) with an access hole (484) and an intracranial monitoring device (480) shaped and dimensioned for passage through the access hole (484) of the intracranial monitoring device recess (482).

3. The cerebral spinal fluid shunt plug (410, 710) according to claim 1 or 2, wherein the window is optically transparent and/or is optically translucent to all light waves and/or is sonolucent and/or is radiolucent.

4. The cerebral spinal fluid shunt plug (410, 710) according to claim 1 to 3, wherein the window is optically transparent, optically translucent to all light waves, is sonolucent, and is radiolucent.

5. The cerebral spinal fluid shunt plug (410, 710) according to claim 1 to 4, wherein the window comprises polymethyl methacrylate (PMMA).

6. The cerebral spinal fluid shunt plug (410, 710) according to claim 1 to 5, wherein the window is a lucent disk (780), wherein the lucent disk (780) preferably includes an upper surface and a lower surface and the curvature of the upper surface differs from the curvature of the lower surface or the lucent disk (780) preferably includes an alignment feature.

7. The cerebral spinal fluid shunt plug (410, 710) according to claim 6, wherein the alignment feature includes a series of markings at different depths within the lucent disk (780).

8. The cerebral spinal fluid shunt plug (410, 710) according to claim 7, wherein the series of markings includes an outer first lucent disk (780) marking and an inner second lucent disk (780) marking formed along the upper and lower surfaces, respectively, of the lucent disk (780), wherein the series of markings preferably further includes an interior lucent disk (780) marking formed within a body of the lucent disk (780) and in alignment with the outer first lucent disk (780) marking and the inner second lucent disk (780) marking.

9. The cerebral spinal fluid shunt plug (410, 710) according to claim 6, wherein the lucent disk (780) includes channels.

10. The cerebral spinal fluid shunt plug (410, 710) according to claim 1 to 9, wherein the intracranial monitoring device (480) is a wireless intracranial monitoring device.

11. The cerebral spinal fluid shunt plug (410, 710) according to claim 10, wherein the intracranial monitoring device (480) includes a probe that passes through the access hole.

## Patentansprüche

1. Shuntstopfen (410, 710) für Gehirn-Rückenmarks-Flüssigkeit, umfassend:
ein Shuntstopfengehäuse (418, 718) aufweisend eine obere Fläche (432, 732), eine untere Fläche (434), durchgehende Seitenwände, die sich zwischen der oberen Fläche (432, 732) und der unteren Fläche (434) sowie um den Umfang des Shuntstopfengehäuses erstrecken, eine Shuntventilaussparung (438, 738), die in der oberen Fläche (432, 732) des Shuntstopfengehäuses (418, 718) ausgebildet ist, und entweder eine intrakranielle Überwachungsaussparung (482) mit einer Zugangsöffnung (484) oder eine Fensteraussparung (782) mit einer mittigen Zugangsöffnung (784), die in der oberen Fläche (432, 732) des Shuntstopfengehäuses (418, 718) ausgebildet sind,
wobei das Shuntstopfengehäuse (418, 718) ferner Durchgänge umfasst, die eine kommunizierende Verbindung der Shuntventilaussparung (438, 738) mit einem Äußeren des Shuntstopfengehäuses ermöglichen, wobei die Zugangsöffnungen (484, 784) oder die Durchgänge so geformt und bemessen sind, um eine Verbindung eines Ventrikelkatheters und eines Peritonealkatheters mit dem innerhalb der Aussparung (438, 738) des Shuntstopfengehäuses (418, 718) untergebrachten Shuntventil zu ermöglichen, wobei das Gehäuse (418, 718) zum Positionieren innerhalb eines ärztlich geformten Schädellochs (500) auf eine Weise geformt und bemessen ist, die es ermöglicht, dass die obere Fläche (432, 732) des Gehäuses (418, 718) im Wesentlichen bündig mit einer Schädel-Außenfläche ist und ein Vorsprung (434p, 734p) entlang der unteren Fläche innerhalb des Schädellochs positioniert ist, und
ein Fenster oder eine intrakranielle Überwachungsvorrichtung (480), die zum jeweiligen Positionieren innerhalb der Fensteraussparung (782) oder der intrakraniellen Überwachungsaussparung (482) des Shuntstopfengehäuses (418, 718) geformt und bemessen sind.

2. Shuntstopfen (410, 710) für Gehirn-Rückenmarks-Flüssigkeit nach Anspruch 1, wobei die Aussparung mit einer Zugangsöffnung eine Fensteraussparung (782) mit einer Zugangsöffnung (784) ist und der Shuntstopfen (410, 710) für Gehirn-Rückenmarks-Flüssigkeit das Fenster aufweist, das zum Positionieren innerhalb der Fensteraussparung (782) geformt und bemessen ist, wobei der Shuntstopfen (410, 710) für Gehirn-Rückenmarks-Flüssigkeit ferner eine intrakranielle Überwachungsvorrichtungsaussparung (482) mit einer Zugangsöffnung (484) und eine intrakranielle Überwachungsvorrichtung (480), die für einen Durchgang durch das Zugangsloch (484) der intrakraniellen Überwachungsvorrichtungsaussparung (482) geformt und bemessen ist, umfasst.

3. Shuntstopfen (410, 710) für Gehirn-Rückenmarks-Flüssigkeit nach Anspruch 1 oder 2, wobei das Fenster optisch durchsichtig ist und/oder optisch durchscheinend für alle Lichtwellen ist und/oder schalldurchlässig ist und/oder strahlendurchlässig ist.

4. Shuntstopfen (410, 710) für Gehirn-Rückenmarks-Flüssigkeit nach einem der Ansprüche 1 bis 3, wobei das Fenster optisch durchsichtig ist, optisch durchscheinend für alle Lichtwellen, schalldurchlässig ist und strahlendurchlässig ist.

5. Shuntstopfen (410, 710) für Gehirn-Rückenmarks-Flüssigkeit nach einem der Ansprüche 1 bis 4, wobei das Fenster Polymethylmethacrylat (PMMA) umfasst.

6. Shuntstopfen (410, 710) für Gehirn-Rückenmarks-Flüssigkeit nach einem der Ansprüche 1 bis 5, wobei das Fenster eine klare Scheibe (780) ist, wobei die klare Scheibe (780) vorzugsweise eine obere Fläche und eine untere Fläche umfasst und sich die Krümmung der oberen Fläche von der Krümmung der unteren Fläche unterscheidet oder die klare Scheibe (780) vorzugsweise ein Ausrichtungsmerkmal aufweist.

7. Shuntstopfen (410, 710) für Gehirn-Rückenmarks-Flüssigkeit nach Anspruch 6, wobei das Ausrichtungsmerkmal eine Reihe von Markierungen bei unterschiedlichen Tiefen innerhalb der klaren Scheibe (780) umfasst.

8. Shuntstopfen (410, 710) für Gehirn-Rückenmarks-Flüssigkeit nach Anspruch 7, wobei die Reihe von Markierungen eine äußere erste Markierung der klaren Scheibe (780) und eine innere zweite Markierung der klaren Scheibe (780) umfasst, die jeweils entlang der oberen beziehungsweise der unteren Fläche der klaren Scheibe (780) geformt sind, wobei die Reihe von Markierungen vorzugsweise ferner eine interne Markierung der klaren Scheibe (780) umfasst, die innerhalb eines Körpers der klaren Scheibe (780) ausgebildet ist und mit der äußeren ersten Markierung der klaren Scheibe (780) und der inneren zweiten Markierung der klaren Scheibe (780) gleichgerichtet ist.

9. Shuntstopfen (410, 710) für Gehirn-Rückenmarks-Flüssigkeit nach Anspruch 6, wobei die klare Scheibe (780) Kanäle umfasst.

10. Shuntstopfen (410, 710) für Gehirn-Rückenmarks-Flüssigkeit nach einem der Ansprüche 1 bis 9, wobei die intrakranielle Überwachungsvorrichtung (480) eine drahtlose intrakranielle Überwachungsvorrichtung ist.

11. Shuntstopfen (410, 710) für Gehirn-Rückenmarks-Flüssigkeit nach Anspruch 10, wobei die intrakranielle Überwachungsvorrichtung (480) eine Sonde umfasst, die durch die Zugangsöffnung hindurchgeht.

## Revendications

1. Bouchon de shunt de liquide céphalo-rachidien (410, 710), comprenant:
un logement de bouchon de shunt (418, 718) incluant une surface supérieure (432, 732), une surface inférieure (434), des parois latérales continues s'étendant entre la surface supérieure (432, 732) et la surface inférieure (434), ainsi qu'autour de la périphérie du logement de bouchon de shunt, un creux pour valve de shunt (438, 738) étant formé dans la surface supérieure (432, 732) du logement de bouchon de shunt (418, 718) et soit un creux pour surveillance intracrânienne (482) avec un trou d'accès (484) soit un creux pour fenêtre (782) avec un trou d'accès central (784) étant formé dans la surface supérieure (432, 732) du logement de bouchon de shunt (418, 718), le logement de bouchon de shunt (418, 718) incluant en outre des passages permettant au creux pour valve de shunt (438, 738) de communiquer avec un extérieur du logement de bouchon de shunt, les trous d'accès (484, 784) ou des passages étant formés et dimensionnés pour permettre la connexion d'un cathéter ventriculaire et d'un cathéter péritonéal avec la valve de shunt logée à l'intérieur du creux (438, 738) du logement de bouchon de shunt (418, 718), le logement (418, 718) étant formé et dimensionné pour le positionnement à l'intérieur d'un trou crânien (500) formé par un médecin de manière à permettre à la surface supérieure (432, 732) du logement (418, 718) d'être essentiellement à l'affleur avec une surface extérieure d'un crâne et une saillie (434p, 734p) le long de la surface inférieure étant positionnée à l'intérieur du trou crânien ; et
une fenêtre ou un dispositif de surveillance intracrânienne (480) formés et dimensionnés pour le positionnement respectivement à l'intérieur de soit le creux pour fenêtre (782) soit le creux pour surveillance intracrânienne (482) du logement de bouchon de shunt (418, 718).

2. Bouchon de shunt de liquide céphalo-rachidien (410, 710) selon la revendication 1, dans lequel le creux avec un trou d'accès est un creux pour fenêtre (782) avec un trou d'accès (784) et le bouchon de shunt de liquide céphalo-rachidien (410, 710) inclut la fenêtre formée et dimensionnée pour le positionnement à l'intérieur du creux pour fenêtre (782), le bouchon de shunt de liquide céphalo-rachidien (410, 710) incluant en outre un creux pour dispositif de surveillance intracrânienne (482) avec un trou d'accès (484) et un dispositif de surveillance intracrânienne (480) formé et dimensionné pour le passage à travers le trou d'accès (484) du creux pour dispositif de surveillance intracrânienne (482).

3. Bouchon de shunt de liquide céphalo-rachidien (410, 710) selon la revendication 1 ou 2, dans lequel la fenêtre est optiquement transparente et/ou est optiquement translucide à toutes les ondes lumineuses, et/ou est sonolucide et/ou est radiolucide.

4. Bouchon de shunt de liquide céphalo-rachidien (410, 710) selon la revendication 1 à 3, dans lequel la fenêtre est optiquement transparente, optiquement translucide à toutes les ondes lumineuses, est sonolucide et est radiolucide.

5. Bouchon de shunt de liquide céphalo-rachidien (410, 710) selon la revendication 1 à 4, dans lequel la fenêtre comprend du polyméthyle méthacrylate (PMMA).

6. Bouchon de shunt de liquide céphalo-rachidien (410, 710) selon la revendication 1 à 5, dans lequel la fenêtre est un disque lucide (780), dans lequel le disque lucide (780) inclut de préférence une surface supérieure et une surface inférieure et la courbure de la surface supérieure diffère de la courbure de la surface inférieure ou bien le disque lucide (780) inclut de préférence une caractéristique d'alignement.

7. Bouchon de shunt de liquide céphalo-rachidien (410, 710) selon la revendication 6, dans lequel la caractéristique d'alignement inclut une série de marquages à différentes profondeurs à l'intérieur du disque lucide (780).

8. Bouchon de shunt de liquide céphalo-rachidien (410, 710) selon la revendication 7, dans lequel la série de marquages inclut un premier marquage extérieur de disque lucide (780) et un second marquage intérieur de disque lucide (780) formés le long des surfaces supérieure et inférieure, respectivement, du disque lucide (780), dans lequel la série de marquages inclut en outre de préférence un marquage intérieur de disque lucide (780) à l'intérieur d'un corps du disque lucide (780) et en alignement avec le premier marquage extérieur de disque lucide (780) et le second marquage intérieur de disque lucide (780).

9. Bouchon de shunt de liquide céphalo-rachidien (410, 710) selon la revendication 6, dans lequel le disque lucide (780) inclut des canaux.

10. Bouchon de shunt de liquide céphalo-rachidien (410, 710) selon la revendication 1 à 9, dans lequel le dispositif de surveillance intracrânienne (480) est un dispositif de surveillance intracrânienne sans fil.

11. Bouchon de shunt de liquide céphalo-rachidien (410, 710) selon la revendication 10, dans lequel le dispositif de surveillance intracrânienne (480) inclut une sonde qui passe à travers le trou d'accès.
